# EUROPÄISCHE PATENTSCHRIFT

(11) **EP 1 405 123 B1**
(45) Veröffentlichungstag und Bekanntmachung des Hinweises auf die Patenterteilung: **21.03.2007**
(21) Anmeldenummer: 01987895.8
(22) Anmeldetag: 08.10.2001
(51) Int. Cl.: G02B 27/01

(54) **Informationssystem und Verfahren zur Zurverfügungstellen von Informationen unter Verwendung eines Holographischen Elements**
Information system and method for providing information using a holographic element
Systàme d'informations et procédé de diffusion d'informations faisant intervenir l'utilisation d'un element holographique

(30) Priorität: 07.10.2000 WO PCT/EP00/09840; 07.10.2000 WO PCT/EP00/09843; 07.10.2000 WO PCT/EP00/09841; 07.10.2000 WO PCT/EP00/09842; 22.05.2001 WO PCT/EP01/05886; 08.06.2001 DE 10127826
(43) Veröffentlichungstag der Anmeldung: 07.04.2004
(73) Patentinhaber: Dickerson, David, 85354 Freising (DE)
(72) Erfinder: EBERL, Roland, H.C., 82061 München-Neuried (DE); EBERL, Heinrich A., 87463 Probstried (DE); DICKERSON, David, 85354 Freising (DE)
(86) Internationale Anmeldenummer: PCT/EP2001/011633
(87) Internationale Veröffentlichungsnummer: WO 2002/033472

(56) Entgegenhaltungen:
- WO-A-98/13720
- US-A- 5 973 781
- US-A- 6 008 781
- US-A- 6 027 216

## Beschreibung

### Gebiet der Erfindung

Die vorliegende Erfindung betrifft ein Informationssystem sowie ein Verfahren zum Zurverfügungstellen von Informationen unter Verwendung eines holographischen Elements, wobei das Zurverfügungstellen der Informationen insbesondere in Korrelation mit auf ein Auge fattendes Licht erfolgt.

### Stand der Technik

In den Anmeldungen WO 02 31580, WO 02 31577, WO 02 31578, WO 02 315 79, die alle am 07. Oktober 2000 eingereicht wurden, und DE 101 27 826, die am 08. Juni 2001 eingereicht wurde, sind mannigfaltige Informations-, Projektions- und Aufnahmesysteme sowie entsprechende Verfahren beschrieben, die dem Ziel verfolgen, Information in einer den Anforderungen eines sehenden Menschen angepaßten Art und Weise zur Verfügung zu stellen. Schwierig ist es jedoch, ein solches System oder Verfahren derart kostengünstig zu realisieren, daß es einer breiten Öffentlichkeit zugänglich ist.

In der Anmeldung WO02097511 sind mannigfaltige Verfahren und Vorrichtungen zur Anpassung eines optischen Systems an die Blickrichtung des menschlichen Auges und zugehörige Systeme zur Bestimmung der Veränderung der Relativlage zwischen dem optischen System und dem optischen System des Auges beschrieben, die beispielsweise in Zusammenhang mit den oben genannten Informations-, Projektions- und Aufnahmesysteme und entsprechenden Verfahren nützlich sind. Auch hier ist es jedoch schwierig, eine solche Vorrichtung oder Verfahren derart kostengünstig zu realisieren, daß es einer breiten Öffentlichkeit zugänglich ist.

Ähnliche Überlegungen gelten der Vorrichtung zur Aufnahme des Netzhautreflexbildes und. Überlagerung von Zusatzbildern gemäß der Offenlegungsschrift DE 196 31 414 A1 sowie dem Verfahren zur Verbesserung des optischen Wahrnehmungsvermögens durch Modifikation des Netzhautbildes gemäß der Offenlegungsschrift DE 197 28 890 A1.

US 6,252,565 B1 zeigt ein optisches retinales Display auf der Basis eines elliptischen Hohlraums. Dieses Display weist eine unvorteilhaft klobige Form auf.

Näheres zum Stand der Technik enthält US 6,008,781, die ein virtuelles retinales Display beschreibt, das Photonengeneration und -manipulation dazu verwendet, ein panoramisches hochauflösendes virtuelles Farbbild zu erzeugen, das direkt auf die Netzhaut des Auges ohne Erzeugung eines reellen Bildes oder eines Luftbildes, das mittels eines Spiegels oder einer Optik betrachtet wird, projiziert wird.

Es ist eine Aufgabe der vorliegenden Erfindung, die oben genannten Nachteile des Standes der Technik zu überwinden.

### Zusammenfassung der Erfindung

Erfindungsgemäß wird diese Aufgabe durch das Informationssystem gemäß Anspruch 1, das Informationssystem gemäß Anspruch 14, das Verfahren zum Zurverfügungstellen von Informationen gemäß Anspruch 22 oder das Verfahren zum Zurverfügungstellen von Informationen gemäß Anspruch 35 gelöst. Bevorzugte Ausführungsformen der Erfindung sind in den Unteransprüchen beschrieben.

Die beanspruchte Erfindung läßt sich durch die im Folgenden beschriebenen Ausführungsbeispiele eines Informationssystems bzw. eines Verfahrens zum Zurverfügungstellen von Informationen besser verstehen. Im allgemeinen beschreiben die beschriebenen Ausführungsbeispiele bevorzugte Ausführungsbeispiele der Erfindung. Dem aufmerksamen Leser wird jedoch auffallen, daß einige Aspekte der beschriebenen Ausführungsbeispiele über den Schutzumfang der Ansprüche hinausragen. Sofern die beschriebenen Ausführungsbeispiele tatsächlich über den Schutzumfang der Ansprüche hinausragen, sind die beschriebenen Ausführungsbeispiele als zusätzliches Hintergrundinformation zu betrachten und stellen keine Definition der Erfindung per se dar. Entsprechend ist das Wort "erfindungsgemäß" im Folgenden gegebenenfalls als "vorzugsweise" zu verstehen.

In seiner allgemeinsten Form umfaßt das erfindungsgemäße Informationssystem zum Zurverfügungstellen ein holographisches Element und eine optische Abtastvorrichtung, die auf das Auge fallendes Licht über das holographischen Element erfaßt. Ein solches Informationssystem wird als abtastendes Informationssystem bezeichnet.

Alternativ kann das erfindungsgemäße Informationssystem zum Zurverfügungstellen von Informationen in seiner allgemeinsten Form ein holographisches Element und eine optische Projektionsvorrichtung umfassen, die Licht über das holographischen Element in das Auge projiziert. Ein solches Informationssystem wird als projizierendes Informationssystem bezeichnet.

Im Sinne der Erfindung umfaßt der Ausdruck "auf das Auge fallende Licht" sowohl in Richtung Auge einfallendes Licht, das nach Auftreffen auf das Auge von diesem zurückreflektiert worden ist, als auch in Richtung Auge einfallendes Licht, das kurz vor seinem Auftreffen auf das Auge erfaßt oder zur Erfassung abgelenkt und somit vom tatsächlichen Auftreffen auf das Auge abgehalten wird. Das in Richtung Auge einfallendes Licht kann sowohl Umgebungslicht als auch Licht sein, das zum Zwecke der Ausführung der Erfindung aktiv in das Auge eingestrahlt wird.

Erfindungsgemäß wird auf das Auge fallendes Licht über ein holographisches Element erfaßt und/oder Licht über ein holographisches Element in das Auge projiziert. Während die Lichterfassung der Informationsgewinnung dient, kann die Lichtprojektion sowohl der Informationsgewinnung als auch dem Zurverfügungstellen von Informationen dienen. Das holographische Element wird vorzugsweise vor dem Auge gelagert. Da das Auge nicht Teil des erfindungsgemäßen Informationssystems ist, ist diese Aussage dahingehend zu interpretieren, daß die Gestaltung des Informationssystems eine Positionierung des holographischen Elements vor einem Auge erlaubt.

In Anbetracht der Tatsache, daß die erfindungsgemäß unter dem Begriff "Zurverfügungstellen von Informationen" zu verstehenden Möglichkeiten der Informationsgewinnung und des anschließenden Zurverfügungstellens in den oben genannten Anmeldungen sehr ausführlich (einige hundert Seiten) beschrieben sind, wird das erfindungsgemäße "Zurverfügungstellen von Information" neben dem obigen Bezug auf den Inhalt jener Anmeldungen hier pauschal als jegliches Zurverfügungstellen von Informationen definiert, das ein augenbezogenes Zurverfügungstellen von Informationen und/oder ein Zurverfügungstellen von augenbezüglich gewonnenen Informationen umfaßt.

Erfindungsgemäß können die Informationen einem Menschen taktil visuell, hörbar, riechbar und/oder geschmacklich zur Verfügung gestellt werden. Vorzugsweise wird eine Informationsdarbietung ermöglicht, die den Bedürfnissen eines sehenden Menschen auf bisher unerreichte Art und Weise nachkommt. Hierzu kann gehören, daß die Informationen dem Menschen in geeigneter Weise, das heißt unter Ausnutzung eines oder mehrerer der fünf Sinne, zur Verfügung gestellt werden können. Die Informationen können jedoch auf beliebige Art und Weise zur Verfügung gestellt werden und bedürfen keinen bestimmten Adressant. Beispielsweise können die Informationen einem weiteren System elektromagnetisch, mechanisch und/oder optisch zur Verfügung gestellt werden oder durch eine optische oder akustische Ausgabevorrichtung in die Umgebung ausgestrahlt werden.

Der Verständlichkeit halber wird in der Beschreibung bei Bedarf zwischen einem Zurverfügungstellen von Information bzw. einer Informationsgewinnung im primären Sinne der Erfindung und einem Zurverfügungstellen von Information bzw. einer Informationsgewinnung im sekundären Sinne der Erfindung unterschieden. Als ein Zurverfügungstellen von Information bzw. eine Informationsgewinnung im primären Sinne der Erfindung ist "eine Projektion von Licht in ein Auge über ein holographisches Element" bzw. "eine Erfassung von auf ein Auge fallendem Licht über ein holographisches Element" zu verstehen. Jedes andere oder weitere Zurverfügungstellen von Information und/oder jede andere oder weitere Informationsgewinnung wird als ein Zurverfügungstellen von Information bzw. eine Informationsgewinnung im sekundären Sinne der Erfindung bezeichnet.

Erfindungsgemäß umfaßt das holographische Element vorzugsweise eine oder mehrere holographischen Aufnahmen (was einem "Hologramm" entspricht) eines tatsächlichen oder durch direkt oder indirekt computergesteuerte Belichtung des der holographischen Aufnahme zugrundeliegenden Photomaterials virtuell emulierten Gegenstandes und ist somit in der Lage ist, die Brechungs-, Beugungs- und/oder Reflektionseigenschaften dieses Gegenstands in gewissen Maßen unter den bestimmten Umständen der holographischen Wiedergabe nachzuahmen. Insbesondere läßt sich hierdurch Information über die dreidimensionale Topologie des Gegenstands festhalten und wiedergeben.

Dabei von Vorteil ist, daß das holographische Element die darin festgehaltene Topologie selbst nicht in seiner äußerlichen Form aufweisen muß. Beispielsweise könnte ein flaches holographisches Element die Brechungs-, Beugungs- und/oder Reflektionseigenschaften eines gekrümmten Gegenstands emulieren. Ebenfalls könnte ein gekrümmtes holographisches Element die Brechungs- Beugungs- und/oder Reflektionseigenschaften eines andersartig gekrümmten oder flachen Gegenstands emulieren. Ein holographisches Element kann auch bei unterschiedlichen Wellenlängen die Brechungs-. Beugungs- und/oder Reflektionseigenschaften jeweilig verschiedener Gegenstände emulieren. Im folgenden Text wird für diese Eigenschaften das Verb 'brechen' benutzt, auch wenn im Wesentlichen Beugungseffekte zugrunde liegen. Näheres zu diesem Thema findet sich im Abschnitt 2 (,holographisches Element).

Während bei einer üblichen photographischen Aufnahme die Intensität (bei Farbphotographie in Abhängigkeit von der Farbe bzw. Wellenlänge) des auf das Photomaterial fallenden Lichts erfaßt wird, erfaßt das feinkörnige Photomaterial bei einer holographischen Aufnahme das sehr feine Interferenzmuster der auf das Photomaterial fallenden Lichtwellen. Aus diesem Grund hängt die holographische Wiedergabe, die einer Lichtbrechung im Sinne der Erfindung entspricht, im allgemeinen stark von der Wellenlänge, der Einfallswinkel und der Phase des einfallenden Lichtes ab.

Über die Technik der holographischen Aufnahme und Wiedergabe sowie über die damit erzielbaren Möglichkeiten und die jeweiligen Einschränkungen dieser Techniken kann beispielsweise in den Offenlegungsschriften DE 197 03 592 A1 und DE 197 99 162 A1 sowie in den Büchern und Veröffentlichungen "Optical Holography: Principles. Techniques, and Applications (Cambridge Studies in Modern Optics)'' von P. Hariharan (ISBN: 0521439655), "Introduction To Fourier Optics" von Joseph Goodman (ISBN: 0070242542), "Optical Information Processing and Holography" von W. Thomas Cathey, John Wiley & Sons, N.Y., 1974. Computer Generated Holograms: Techniques and applications" von Wai-Hon Lee in E. Wolf, Progress in Optics XVI, 1978 und "Topics in Applied Physics, Vol. 20: Holographic Recording Materials", H.M. Smith. Herausg., Springer-Verlag, Berlin, 1977 und den darin erwähnten Veröffentlichungen nachgelesen werden. Der gesamte Inhalt dieser Bücher und Veröffentlichungen wird deshalb ausdrücklich durch Bezugnahme in diese Anmeldung einbezogen.

Die Ausbreitung von Licht kann über Brechung, Beugung und/oder Reflektion beeinflußt werden, wobei der passende Begriff stark kontextabhängig ist. Im vorhergehenden Abschnitt wurde ersichtlich, daß weder eine namentliche Auslistung der möglichen Ausbreitungsänderungsarten noch der Begriff "Lichtausbreitungsänderung" eine knappe verständliche Formulierung zuläßt. In der Beschreibung wird deshalb der Begriff "Brechung" bzw. "brechen" als synonym mit dem tatsächlichen Oberbegriff "Ausbreitungsänderung" verwendet.

Im Sinne der Erfindung ist eine optische Abtastvorrichtung jede Vorrichtung, die in der Lage ist, optische Signale zum Zwecke der Informationsgewinnung zu detektieren oder zu erfassen. (Im übrigen Teil der Anmeldung wird sowohl das Detektieren von Licht als auch das Erfassen von Licht der Einfachheit halber häufig als "Lichterfassung" bezeichnet.) Üblicherweise umfassen solche Abtastvorrichtung mindestens einen Signalwandler, um eine für eine eventuelle Bearbeitung ausreichende Signaltrennung, Signalstärke oder dergleichen zu gewährleisten. Somit könnte die erfindungsgemäße optische Abtastvorrichtung einen optischen Signalwandler, beispielsweise eine optoelektronische Vorrichtung wie ein CCD-Photoempfänger, eine Photomultiplier-Vorrichtung oder eine Photodiode oder eine rein optische Vorrichtung, die das abgetastete Licht in rein optische Signale für eine weitere optischen und/oder opto-elektronischen Bearbeitung umwandelt. Bei solchen rein optischen Vorrichtungen ist in letzter Zeit weltweit erheblicher technischer Fortschritt erzielt worden, so daß mit ihrem kommerziellen Einsatz auch im Rahmen der Erfindung in nächster Zukunft zu rechnen sei.

Eine optische Projektionsvorrichtung im Sinne der Erfindung ist jede Vorrichtung, die in der Lage ist, optische Signale zu emittieren oder Licht in einer kontrollierbaren Art und Weise abzugeben. (Sowohl das Emittieren von optischen Signalen als auch die kontrollierte Abgabe von Licht wird der Einfachheit halber im übrigen Teil der Anmeldung als "Projektion" bezeichnet.) Zum Ersteren gehören beispielsweise Laser. Laserdioden. LED's, OLED's, etc. Letztere könnte zum Beispiel eine Kombination aus einer Lichtquelle, einem in der Fachsprache häufig als "light valve" (zu deutsch: Lichtventil) bezeichneten Modulator und einer Lichtleitanordnung, die das von der Lichtquelle erzeugte Licht zum Modulator leitet. Vorzugsweise ist die optische Projektionsvorrichtung selbst oder in Zusammenarbeit mit einer weiteren Vorrichtung in der Lage. Licht zu projizieren, das bezüglich seiner Intensität. Ausbreitungsrichtung, Polarisation, spektralen Zusammensetzung, insbesondere seiner Wellenlänge, und/oder eines anderen seiner Kenngrößen bestimmbar ist. Werden die Kenngrößen des Lichtes zeitlich verändert, wird dies in der Fachsprache als "Modulation" bezeichnet.

Die Ausbreitung von Licht wird typischerweise als strahlenartig bezeichnet. Das erfaßte bzw. projizierte Licht wird deshalb in der Anmeldung häufig als ein erfaßter bzw. projizierter "Lichtstrahl" oder auch "Abtaststrahl" bzw. "Projektionsstrahl" bezeichnet. Dies ist insbesondere bei Diskussionen um den Strahlengang, den Strahlendurchmesser, der spektralen Zusammensetzung und ähnlichen Eigenschaften des erfaßten bzw. projizierten Lichtes der Fall, die häufig mit dem Konzept eines Lichtstrahles assoziiert sind.

Zwecks eines Leiten oder eines Gestalten des erfaßten bzw. projizierten Lichtstrahls kann die erfindungsgemäße optische Abtastvorrichtung bzw. Projektionsvorrichtung über eine Lichtleitvorrichtung und/oder eine Lichtgestaltungsvorrichtung verfügen. Beispiele für solche Vorrichtungen, deren Abgrenzung von einander nicht immer eindeutig ist, sind steuerbare und nicht-steuerbare Spiegel, Teilerspiegel, akusto-optische Modulatoren, holographische Elemente, Blenden, Filter, Linsen. Lichtleiter, etc.

Je nach den Rahmenbedingungen der Ausgestaltung können mehrere optischen Signalwandler, optische Projektionsvorrichtungen, Lichtleitvorrichtungen und/oder Lichtgestaltungsvorrichtungen als separate Einheiten oder integrale Einheit miteinander kombiniert werden.

Wie in dieser Beschreibung verdeutlicht wird läßt sich die vorliegende Erfindung in vorteilhafter Weise im Zusammenhang mit den in den eingangs genannten Offenlegungsschriften bzw. Anmeldungen WO 02 31580, WO 02 31577, WO 02 31578, WO 02 31579, DE 101 27 826, WO 02 097511, DE 196 31 414 A1 und DE 197 28 890 A1 beschriebenen Systemen, Vorrichtungen und Verfahren verwenden. Auch im Zusammenhang mit der in der am 8. Oktober 2001 von der Anmelderin dieser Anmeldung eingereichten Anmeldung mit dem Titel "Vorrichtung und Verfahren zur Bestimmung der Orientierung eines Auges" offenbarten Erfindung läßt sich die vorliegende Erfindung in vorteilhafter Weise anwenden. Der gesamte Inhalt dieser Offenlegungsschriften bzw. Anmeldungen wird deshalb ausdrücklich durch Bezugnahme in diese Anmeldung einbezogen. In Anbetracht der bevorzugte Ausführungsform der darin offenbarten Systeme bzw. Vorrichtungen, werden sie nachfolgend der Einfachheit halber als "Brille" bezeichnet. Auch die vorliegende Erfindung läßt sich in dieser Ausführungsform realisieren. Dabei können holographische Elemente anstelle der Brillengläser oder als Beschichtung der Brillengläser realisiert werden.

Das erfindungsgemäße Informationssystem läßt sich unter anderem als tragbares, freistehendes und/oder mehrteiliges System gestalten. Zum Beispiel läßt sich das erfindungsgemäße Informationssystem als sogenanntes "Palm"-Gerät (zu deutsch: in der Handfläche tragbares Gerät), als in einem Helm integriertes Gerät, als freistehende Untersuchungs-. Behandlungs-, Display- oder Bedienungsvorrichtung oder als mehrteiliges System verwirklichen, bei dem moduliertes Infrarotlicht von einem fernen Teil des Systems vom Auge reflektiert und anschließend über ein holographisches Element erfaßt wird, um die Blickrichtung gegenüber dem fernen Teil des Systems ermitteln zu können.

Es werden viele Merkmale der Erfindung im Laufe dieser Beschreibung sowohl im Allgemeinen als auch im engen Zusammenhang eines jeweiligen, konkret dargestellten Ausführungsbeispiels erläutert Selbstverständlich läßt sich jedes einzelnen Merkmal der Erfindung mit jedem anderen Merkmal kombinieren, soweit die resultierende Kombination nicht zu einem für den Fachmann als sofort unsinnig erkennbaren Ergebnis führt. Hierzu gehört die Austauschbarkeit eines im Singular erwähnten Merkmals mit einer jeweiligen Pluralität dieses Merkmals, sofern die Möglichkeit einer solchen Singularität bzw. Pluralität nicht explizit ausgeschlossen wurde. Auch diejenigen Modifikationen und Alternativen der beschriebenen Merkmale und Merkmalskombinationen, die der Fachmann zur Erfindungsgedanke und zum Erfindungsumfang hinzurechnet, gehören ebenfalls zur Erfindung. Diese Aussagen betreffen nicht die Bestimmung des gewerblichen Schutzbereichs dieser Patentanmeldung/Patent sofern einen Schutzbereich nach anwendbarem Recht durch die Ansprüche verliehen wird.

In der vorliegenden Anmeldung werden, wo es zweckmäßig ist. Merkmale eines Verfahrens beschrieben. Dabei ist stets ausdrücklich eine Vorrichtung offenbart, die geeignet ist, das vorgeschlagene Verfahren auszuführen, beispielsweise ein geeignet programmierter Computer. Sensoren, die in der Lage sind, die notwendigen Signale zu liefern, Signalverarbeitungsvorrichtungen, die in der Lage sind, diese Signale geeignet zu verarbeiten, etc.

Es sei auch explizit darauf hingewiesen, daß alle beschriebenen Vorrichtungsmerkmale sich analog in einem entsprechenden Verfahren zum Zurverfügungstellen von Informationen anwenden lassen.

Nachstehend sind einige weiteren bevorzugte Merkmale und Merkmalskombinationen der Erfindung kurz erläutert
- Das erfindungsgemäße Informationssystem ist vorzugsweise dazu geeignet, Informationen in Korrelation mit auf dem Auge fallendem Licht zur Verfügung zu stellen.
   Eine solche Abhängigkeit mit dem auf dem Auge fallendem Licht wird erfindungsgemäß bei der Ermittlung der Informationen, bei dem Zurverfügungstellen der Information oder während beider dieser inhärenten Vorgängen berücksichtigt. Ein Beispiel für eine Berücksichtigung dieser Abhängigkeit bei der Ermittlung der Informationen wäre, daß das Informationssystem die Informationen in Abhängigkeit vom erfaßten Licht, insbesondere in Abhängigkeit von ermittelten Aussagen dieses erfaßten Lichts, aus einer Informationsquelle, beispielsweise eine Datenbank, eine Sensorik, eine Informationsnetzanbindung und/oder eine Auswertevorrichtung, erhält oder von der Auswertevorrichtung ermitteln läßt. Beim Zurverfügungstellen der Informationen kann diese Abhängigkeit zum Beispiel dadurch berücksichtigt werden, daß die Informationen mittels einer Rückprojektion in das Auge auf eine Art und Weise in das gesehene Bild eingeblendet werden, daß ein zeitlicher, farblicher, räumlicher, kontrastbezogener, oder sonstiger sinnvoller Zusammenhang zwischen den Informationen und dem gesehenen Bild hergestellt wird. Insbesondere kann die Abhängigkeit darin bestehen, daß das erfaßte Lichtbild dazu verwendet wird, die Lage und Orientierung des Augapfels festzustellen, so daß ein zwecks eines Zurverfügungstellens der Informationen auf das Auge projiziertes Bild bei einer Bewegung des Auges festzustehen scheint, sich bei einer Bewegung des Auges mitzubewegen scheint oder sich auch bei einer Bewegung des Auges entsprechend einem vorgegebenen Verlauf zu bewegen scheint.
   Durch die bevorzugte Korrelation zwischen dem Zurverfügungstellen von Informationen und dem auf das Auge fallenden Licht wird erreicht, daß der oben erwähnte, vom sehenden Menschen erwartete Zusammenhang zwischen dem Gesehenen und den zur Verfügung gestellten Informationen besteht.
- Vorzugsweise steht die optische Abtastvorrichtung in einem festen Winkelverhältnis zum holographischen Element.
   Gemäß einer Ausführung der Erfindung erfaßt eine optische Abtastvorrichtung auf das Auge fallendes Licht über ein holographisches Element. Sollen dabei die holographischen Eigenschaften des holographischen Elements in erfindungsgemäßer vorteilhafter Weise zum Tragen kommen, so müssen unter anderem die oben erwähnten holographischen Winkelbedingungen erfüllt sein. Hierzu kann ein festes Winkelverhältnis zwischen der optischen Abtastvorrichtung und dem holographischen Element beitragen.
   Zur Erzielung dieses Merkmals können die optische Abtastvorrichtung und das holographische Element beispielsweise auf einem einheitlichen festen Gestell, z.B. ein festes Brillengestell, oder auf einem mehrteiligen beweglichen Gestell montiert sein, dessen relevanten Teile in einem festen, vorzugsweise vorbestimmten Verhältnis zueinander fixierbar sind.
- Vorzugsweise erfaßt das optischen Abtastvorrichtung Licht, das von dem holographischen Element vor seinem Auftreffen auf das Auge gebrochen wird und nicht ins Auge gelangt.
   Das Auge ist ein hochwertiges aber kein fehlerfreies optisches System. Erfaßt das erfindungsgemäße Informationssystem beispielsweise Bilder der Umgebung über das Auge als teilreflektierendes Element, so weisen diese Bilder Verzeichnungsfehler auf. Wird Licht vor seinem Auftreffen auf das Auge vom holographischen Element gebrochen, so wird dieses Licht nicht vom optischen System des Auges beeinflußt.
- Vorzugsweise erfaßt das optischen Abtastvorrichtung Licht, das erst von dem Auge zurückreflektiert und dann vom holographischen Element gebrochen worden ist.
   In vielen Anwendungen ist es sinnvoll, Licht zu erfassen, das vom Auge zurückreflektiert worden ist. Beispielsweise könnte das erfindungsgemäße Informationssystem ein retinales Reflexbild des auf die Retina eines Auges einfallenden Umgebungslichtes im sichtbaren Spektralbereich gleichzeitig mit einem Bild der im Infrarotbereich abstrahlenden Netzhautstrukturen abtasten, um zu erfahren, auf welches Gebiet der Umgebung der Bereich des schärfsten Sehens des Auges momentan gerichtet ist.
   Eine optische Abtastvorrichtung zur Erfassung von Licht aus dem Auge kann durch eine entsprechend ausgestaltete, vorzugsweise vor dem Auge gelagerte Lichtleitvorrichtung, die das Licht beispielsweise zur Erfassung bündelt, vereinfacht werden. Die Benutzung eines holographischen Elements als lichtleitendes Element zur Brechung von aus dem Auge zurückreflektiertern Licht ermöglicht eine kostengünstige Realisierung einer solchen, üblicherweise komplexen Lichtleitvorrichtung.
- Vorzugsweise bricht das holographische Element aus dem Gesichtsfeld des Auges stammendes Licht lediglich bei einigen diskreten Wellenlängen im sichtbaren Bereich vor seinem Auftreffen auf das Auge zur Erfassung durch die optische Abtastvorrichtung und bricht von dem Auge zurückreflektiertes Licht lediglich bei einer diskreten Wellenlänge im Infrarotbereich zur Erfassung durch die optische Abtastvorrichtung.
   Eine solche Ausgestaltung der Erfindung wäre gemäß den obigen Ausführungen zum Beispiel sinnvoll, um sowohl ein verhältnismäßig verzeichnungsfreies Bild der Umgebung als auch ein beispielsweise zur Erfassung der Blickrichtung wertvolles Bild der im Infrarotbereich abstrahlenden Netzhautstrukturen erfassen zu können. In Anbetracht der Tatsache, daß die Kornea Infrarotstrahlung sehr gut reflektiert, ist eine solche Anordnung auch zur Erfassung von Korneareflexbildern sehr sinnvoll.
   Durch die Beschreibung hindurch weisen Angaben über beispielsweise "lichtbrechende Eigenschaften des holographischen Elements bei einer oder einigen diskreten Wellenlängen" auf die Möglichkeit hin, die Wellenlänge(n) des von der optischen Projektionsvorrichtung projizierten Lichts bzw. die bevorzugt erfaßten Wellenlänge(n) der optischen Abtastvorrichtung an die Lichtbrechungseigenschaften des holographischen Elements anzupassen, oder umgekehrt. Zudem könnten die Lichtbrechungseigenschaften des holographischen Elements an die Anwendungserfordernisse angepaßt sein. Beispielsweise im Rahmen einer Ausgestaltung des erfindungsgemäßen Informationssystems als Nachtsichtbrille wäre es vorteilhaft, wenn das holographische Element aus dem dem Auge zugeordneten Gesichtsfeld stammendes Licht bei einer im Infrarotbereich liegenden diskreten Wellenlänge vor seinem Auftreffen auf das Auge und/oder nach seiner Reflektion vom Auge in Richtung einer Abtastvorrichtung brechen würde, Im Fall einer Vollfarbprojektion wäre es angebracht, wenn das holographische Element in der Lage wäre, das von der Projektionsvorrichtung projizierte, vorzugsweise rote, blaue und grüne Licht in Richtung Auge zu brechen.
   Im Falle einer genauen Abstimmung der WeJlenlängen des zu brechenden Lichtes und der lichtbrechenden Eigenschaften des holographischen Elements kann zum Beispiel auch erreicht werden, daß das holographische Element trotz hoher Brechungswirkung bei den zu brechenden Wellenlängen Licht anderer Wellenlängen im wesentliche ungestört passieren läßt. Dies ist insbesondere dann von Vorteil, wenn das holographische Element vor dem Auge gelagert ist, da die Wahrnehmung des Gesichtsfeld ansonsten beeinträchtigt werden könnte.
- Vorzugsweise bricht das holographische Element aus dem Gesichtsfeld des Auges stammendes Licht bei weniger als 20, weniger als 10 oder weniger als 5 diskreten Wellenlängen im sichtbaren Bereich entweder vor seinem Auftreffen auf das Auge oder nach seiner Rückstreuung aufgrund des Auges zur Erfassung durch die optische Abtastvorrichtung.
   Wie oben besprochen, beeinflußt ein holographisches Element nur das darauf fallende Licht, das die oben erwähnten holographischen Kriterien erfüllt. Derzeit ist es schwierig, Hologramme herzustellen, die Licht über einen breiten Spektralbereich beeinflussen. Die Erfindung sieht deshalb bevorzugt vor, daß das holographische Element aus dem Gesichtsfeld des Auges stammendes Licht lediglich bei einigen diskreten Wellenlängen im sichtbaren Spektralbereich bricht. Ein solches Hologramm läßt sich mit geringer Aufwand, beispielsweise durch bei den relevanten Wellenlängen entsprechend wiederholte Beleuchtung, herstellen. Die Auswirkungen der aufgrund der eingeschränkten spektralen Transmissivität des lichtleitenden Hologramms beschränkten Lichterfassung durch entsprechende Anpassung des Informationssystems an die erfaßbaren Wellenlängen kompensieren bzw. reduzieren. Zum Beispiel könnte unter anderem durch entsprechende Bearbeitung der aus dem erfaßten Licht gewonnenen Signalen im Informationssystem, analog dem Prinzip eines Flachbettscanners, der lediglich gefiltertes rotes, grünes und blaues Licht erfaßt, ein hochwertiges Farbbild der Umgebung gewonnen werden. Da ein Hologramm in Analogie üblicherweise ein sehr schmalbandiges Filter darstellt, kann es sinnvoll sein, wenn das holographische Element Licht bei mehr als den üblichen drei (rot, grün, blau) Wellenlängen in Richtung Abtastvorrichtung bricht um beispielsweise aus dem erfaßten Licht ein möglichst naturgetreues Bild gewinnen zu können. Werden jedoch zu viele holographische Aufnahmen in einem einzigen Hologramm festgehalten, so führt dies zu einer gegenseitigen Beeinträchtigung deren Wirkung.
- Vorzugsweise bricht das holographische Element aus dem Gesichtsfeld des Auges stammendes Licht bei einer diskreten Wellenlänge im Infrarotbereich entweder vor seinem Auftreffen auf das Auge oder nach seiner Rückstreuung aufgrund des Auges zur Erfassung durch die optische Abtastvorrichtung.
   Eine Erfassung von Infrarotlicht aus dem Gesichtsfeld ist beispielsweise für Anwendungen interessant, bei denen eine Erfassung von Bilder aus dem Gesichtsfeld bei Dunkelheit, Dämmerung, Regen, etc. erfolgen soll, zum Beispiel bei einer Ausgestaltung des erfindungsgemäßen Informationssystems als Nachtsichtbrille oder Fahrerassistenzsystem.
- Vorzugsweise bricht das holographische Element von dem Auge zurückreflektiertes Licht lediglich bei einer diskreten Wellenlänge im Infrarotbereich zur Erfassung durch die optische Abtastvorrichtung.
   Eine solche Ausgestaltung des erfindungsgemäßen Informationssystems ist zum Beispiel als Eyetracker-System sinnvoll, bei dem die Orientierung des Auges anhand eines erfaßten infraroten Korneareflexbildes oder anhand eines erfaßten Bildes der im Infrarotbereich abstrahlenden Netzhautstrukturen ermittelt wird.
- Vorzugsweise bricht das holographische Element Licht einer oder einigen diskreten Wellenlängen, bei der die optische Abtastvorrichtung eine starke Empfindlichkeit aufweist.
   Durch eine solche Ausgestaltung des erfindungsgemäßen Informationssystems kann erreicht werden, daß möglichst viel des auf das holographische Element fallenden Lichtes von der optischen Abtastvorrichtung erfaßt wird. Es wird also die optische Effizienz des lichterfassenden Teils des Informationssystems gesteigert. Da die Netzhaut nur einen geringen Prozentsatz des darauf fallenden Lichts reflektiert, wäre diese Ausgestaltung beispielsweise bei einer Abtastung eines Netzhautreflexbildes vorteilhaft.
   Bricht das holographische Element lediglich Licht einer oder einigen diskreten Wellenlängen, bei der die optische Abtastvorrichtung eine starke Empfindlichkeit aufweist, kann zum Beispiel erreicht werden, daß das holographische Element trotz hoher Brechungswirkung bei den zu brechenden Wellenlängen Licht anderer Wellenlängen im wesentliche ungestört passieren läßt. Dies ist insbesondere dann von Vorteil, wenn das holographische Element vor dem Auge gelagert ist, da die Wahrnehmung des Gesichtsfeld ansonsten beeinträchtigt werden könnte.
- Vorzugsweise bricht das holographische Element Licht bei einigen diskreten Wellenlängen derart, daß das gebrochene Licht auf einen gemeinsamen Punkt gelenkt wird, und der Einfallswinkel des Lichtes auf diesen Punkt einen eindeutigen, wahlweise auch wellenlängenunabhängigen Rückschluß auf den Einfallswinkel des Lichtes auf das holographische Element erlaubt.
   Wie oben besprochen, läßt sich die Gestaltung der optischen Abtastvorrichtung und/oder weiterer Lichtleitvorrichtungen im allgemeinen vereinfachen, wenn das holographische Element dazu beiträgt, das in Richtung Auge einfallende Licht zu bündeln. Besonders vorteilhaft ist, wenn das gebrochenen Licht auf einen für Licht aller gebrochenen Wellenlängen gemeinsamen Punkt gelenkt wird. An diesem Punkt könnte dann zum Beispiel ein optische Mittelpunkt der optischen Abtastvorrichtung oder einer Scanvorrichtung angeordnet sein. Es ist allerdings vorteilhaft, wenn zurverfügungstehende Information über den Einfallswinkel des Lichtes auf das holographische Element bei der Brechung bzw. Bündelung des Lichtes nicht verloren geht, damit beispielsweise ein Bild, welches typischerweise eine Zuordnung zwischen einem räumlichen Bereich und der Intensität des aus diesem Bereich stammenden Lichtes umfaßt, aus dem erfaßten Licht gewonnen werden kann. Unterliegt die zurverfügungstehende Information über den Einfallswinkel des Lichtes auf das holographische Element keine wellenlängenabhängigen Änderung, so ist es für das erfindungsgemäße Informationssystem einfacher, diese Information gegebenenfalls auszuwerten.
   Näheres zu dem Aufbau eines derartigen holographischen Elements folgt in der nach Themengebieten unterteilten Besprechung der erfindungsgemäßen Merkmale.
- Wie oben schon angesprochen wurde, weist das erfindungsgemäße Informationssystem vorzugsweise eine optische Projektionsvorrichtung auf, die Licht über das holographischen Element in das Auge projiziert.
   Demgemäß kann auch ein abtastendes Informationssystem mit einer optischen Projektionsvorrichtung ausgestattet sein, um beispielsweise sowohl ein optisches Abtasten der Netzhautstrukturen zwecks Eyetracking, d.h. eine Bestimmung der Orientierung des Auges, als auch eine optische Projektion von Bildinformationen ins das Auge zu ermöglichen.
   Ein projizierendes Informationssystem kann eine oder mehrere optischen Projektionsvorrichtungen aufweisen, um beispielsweise Licht in beide Augen eines Benutzers oder verschiedene Lichtstrahlen in das Auge zu projizieren,
- Vorzugsweise läuft das von einer optischen Abtastvorrichtung erfaßte und das vor einer optischen Projektionsvorrichtung projizierte Licht in entgegengesetzter Richtung durch eine gemeinsame Lenkoptik läuft und derart durch die optische Abtastvorrichtung bzw. Projektionsvorrichtung fokussierbar ist, daß ihre jeweiligen Strahlen den gleichen Pfad vom bzw. ins Auge beschreiben.
   Die Benutzung einer gemeinsamen Lichtleitoptik stellt im Regelfall eine Systemvereinfachung dar, da es typischerweise sowohl für eine Reduktion der Anzahl der Komponenten als auch für einen gleichbleibenden Zusammenhang zwischen dem Abtast- und dem Projektionsstrahl sorgt. Zum Beispiel könnte auf diese Art und Weise gewährleistet werden, daß ein von der Retina reflektierter Abtaststrahl aus dem Gebiet der Netzhaut erfaßt wird, das ein Projektionsstrahl bei gleicher Stellung der Lichtleitoptik bestrahlt.
- Vorzugsweise projiziert die optische Projektionsvorrichtung Licht lediglich bei einer oder einigen diskreten Wellenlängen im sichtbaren Bereich und/oder bei einer Wellenlänge im Infrarotbereich.
   Analog dem Prinzip eines Elektrodenstrahlfarbbildschirms, bei dem das Phosphoreszieren von Pixeln in den Grundfarben Rot, Grün und Blau ein Vollfarbbild ermöglicht, könnte beispielsweise die Projektion von verschiedenfarbigem Licht bei einigen diskreten Wellenlängen im sichtbaren Bereich in das Auge die Wahrnehmung eines Vollfarbbilds ermöglichen. Die Projektion von Infrarotlicht könnte zum Beispiel verwendet werden, um Strukturen des Auges zwecks Abtastung zu illuminieren, ohne ein wahrnehmbares, eventuell störendes Bild zu erzeugen.
- Vorzugsweise bricht das holographische Element die Wellenlängen des projizierten Lichtes.
   Eine hohe optische Effizienz des erfindungsgemäßen Informationssystem kann dadurch erreicht werden, daß die Wellenlängen des von der Projektionsvorrichtung projizierten Lichtes gemäß der lichtbrechenden Eigenschaften des holographischen Elements gewählt werden, oder umgekehrt. Wie oben besprochen kann durch eine genaue Abstimmung der Wellenlängen des zu brechenden Lichtes und der lichtbrechenden Eigenschaften des holographischen Elements erreicht werden, daß das holographische Element trotz hoher Brechungswirkung bei den zu brechenden Wellenlängen Licht anderer Wellenlängen im wesentliche ungestört passieren läßt.
   Verfügt das erfindungsgemäße Informationssystem über keine optische Abtastvorrichtung oder erfaßt die optische Abtastvorrichtung kein Licht über das holographische Element, kann es insbesondere vorteilhaft sein, wenn das holographische Element lediglich Licht bei den Wellenlängen des projizierten Lichtes bricht.
- Vorzugsweise steht die optische Projektionsvorrichtung in einem festen, vorbestimmten Winkelverhältnis zum holographischen Element.
   Gemäß einer Ausführung der Erfindung projiziert eine optische Projektionsvorrichtung Licht über ein holographisches Element auf das Auge. Sollen dabei die holographischen Eigenschaften des holographischen Elements in erfindungsgemäßer vorteilhafter Weise zum Tragen kommen, so müssen unter anderem die oben erwähnten holographischen Winkelbedingungen erfüllt sein. Hierzu kann ein festes Winkelverhältnis zwischen der optischen Projektionsvorrichtung und dem holographischen Element beitragen.
   Zur Erzielung dieses Merkmals können die optische Projektionsvorrichtung und das holographische Element beispielsweise auf einem einheitlichen festen Gestell, z.B. ein festes Brillengestell, oder auf einem mehrteiligen beweglichen Gestell montiert sein, dessen relevanten Teile in einem festen, vorzugsweise vorbestimmten Verhältnis zueinander fixierbar sind.
- Das holographische Element umfaßt eine oder mehrere optische Markierungen, deren Lichtreflektionseigenschaften das Informationssystem mittels einer Photodetektorvorrichtung dazu heranziehen kann, einen Projektionswinkel der optischen Projektionsvorrichtung und/oder einer Lichtleitvorrichtung zu eichen.
   Viele Anwendungen der vorliegenden Erfindung, insbesondere beispielsweise die quasi-direkte Projektion einer Bildsequenz auf die Netzhaut über das holographische Element, erfordern eine hohe Projektionsgenauigkeit. Wird die Richtung des Projektionsstrahls durch die optische Projektionsvorrichtung und/oder eine Lichtleitvorrichtung zeitlich geändert, so ist es in vielen Fällen zweckmäßig, wenn ein Vergleich des Ist-Wertes der Projektionsrichtung mit dem Soll-Wert der Projektionsrichtung nach Bedarf möglich ist. Zur Bestimmung des Ist-Wertes der Projektionsrichtung können vorbestimmte optische Markierungen sinnvoll herangezogen werden, deren optischen Eigenschaften beispielsweise das projizierte Licht derart verändern, daß Rückschlüsse auf den Ist-Wert der Projektionsrichtung bezüglich einer, zwei oder drei Dimensionen über die Detektion des veränderten Lichtes möglich ist. Je nach Beteiligung einer Lichtleitvorrichtung an der Bestimmung der Projektionsrichtung liefern die Rückschlüsse auf den Ist-Wert der Projektionsrichtung Rückschlüsse auf einen Projektionswinkel der optischen Projektionsvorrichtung und/oder einer Lichtleitvorrichtung. Dies ermöglicht in bekannter Weise beispielsweise die Eichung der Stellung einer Lichtleitvorrichtung. Wie oben angedeutet, könnte selbstverständlich anstelle des projizierten Lichtes auch des Licht einer anderen Lichtquelle in analoger Weise zur Bestimmung des Projektionswinkels bzw. der Stellung einer Lichtleitvorrichtung.
   Solche optische Markierung, die beispielsweise reflektierende, lichtbrechende und/oder lichtabsorbierende Eigenschaften und eine beliebige ein-, zwei- oder dreidimensionale geometrische Form aufweisen können, werden im holographischen Element verwirklicht, Bei entsprechender Ausführung ist eine holographische Realisierung einer Markierung im holographischen Element kostengünstig, optisch hochwertig und leicht reproduzierbar. Des weiteren kann eine im holographischen Element realisierte Markierung winkel- und/oder wellenlängenselektiv ausgeführt sein. So könnten die Markierungen zum Beispiel lediglich Infrarotlicht brechen, so daß sie für ein Benutzer des erfindungsgemäßen Informationssystems nicht wahrnehmbar sind.
- Vorzugsweise zieht das Informationssystem die Lichtreflektionseigenschaften der optischen Markierungen dazu heran, einen Abtastwinkel der optischen Abtastvorrichtung und/oder einer Lichtleitvorrichtung zu eichen.
   Analog den obigen Ausführungen können optische Markierungen zur Eichung eines Abtastwinkels der optischen Abtastvorrichtung und/oder einer Lichtleitvorrichtung herangezogen werden. Hierzu kann es zweckmäßig sein, eine Quelle vorbestimmten Lichtes vorzusehen, dessen Licht über die optischen Markierungen von der optischen Abtastvorrichtung anstelle eines Detektors erfaßt werden kann.
- Vorzugsweise werden die optischen Markierungen dadurch erzeugt, daß spiegelnde Elemente bei der Erstellung des holographischen Elements derart im holographischen Element abgebildet werden, daß sie Licht einer oder mehreren Wellenlängen, das entsprechend dem vorbestimmten Winkelverhältnis zur optischen Projektionsvorrichtung auf das holographische Element fällt, entlang dem Einfallspfad zurückstrahlt werden.
   Zwecks der einfachen Detektion des von den optischen Markierungen beeinflußten Lichtes ist es vorteilhaft, wenn die Markierungen das aus einer bestimmten Lichtquelle stammende Licht auf einen gemeinsamen Punkt lenken. Somit ist es insbesondere bei Ausgestaltungen der Erfindung, bei denen die Projektionsvorrichtung als Punktlichtquelle betrachtet werden kann, vorteilhaft, wenn die optischen Markierung zumindest teilweise das aus der Richtung der Projektionsvorrichtung stammende Licht zurück entlang dem Einfallspfad strahlt. So könnte dieses zurückgestrahlte Licht dann von einer konfokal zur Projektionsvorrichtung angeordneten Detektorvorrichtung, gegebenenfalls der optischen Abtastvorrichtung, erfaßt werden.
- Vorzugsweise weist die Photodetektorvorrichtung einen Teilerspiegel auf, der derart im Lichtstrahl der optischen Projektionsvorrichtung angeordnet ist, daß er einen Teil des Lichtes, das entgegen der Projektionsrichtung auf den Teilerspiegel trifft, in Richtung eines Photodetektors lenkt, der in mindestens zwei konzentrisch umeinander liegenden Bereiche detektiert.
   Eine konfokale Anordnung der optischen Projektionsvorrichtung und einer Photodetektorvorrichtung, gegebenenfalls der optischen Abtastvorrichtung, läßt sich auf besonders einfache Art und Weise dadurch realisieren, daß ein Teilerspiegel in symmetrischer Weise unmittelbar vor der optischen Projektionsvorrichtung und der Photodetektorvorrichtung angeordnet wird.
   Durch eine Detektoranordnung, die in mindestens zwei konzentrisch umeinander liegenden Bereiche detektiert, läßt sich feststellen, ob ein darauffallender Lichtstrahl sich in Richtung Detektormitte oder von der Detektormitte hinweg bewegt. Dies kann für die Eichung der lichtleitenden Komponenten des Informationssystems vorteilhaft eingesetzt werden.
- Vorzugsweise weist das holographische Element lichtbrechende Eigenschaften bei einer oder einigen diskreten Wellenlängen auf, die einer Spiegelung an der Konkavseite einer gemäß der Krümmung eines rotationssymmetrischen Ellipsoids ausgebildeten Fläche entsprechen.
   Wird das Auge als idealisiertes optisches System betrachtet, bei dem einfallendes Licht stets durch einen gemeinsamen optischen Mittelpunkt verläuft, und werden der optische Ausgang einer Projektionsvorrichtung bzw. einer daran gekoppelten Lichtleitvorrichtung und/oder der Eingang einer Abtastvorrichtung bzw. einer daran gekoppelten Lichtleitvorrichtung als Punktlichtquelle bzw. Punktdetektor betrachtet, so verlaufen von einem der Punkte ausgehende Strahlen stets zum anderen Punkt, wenn diese Punkte den jeweiligen Brennpunkten eines innenreflektierenden rotationssymmetrischen Ellipsoids entsprechen.
   Mit einem holographischen Element lassen sich die Reflektionseigenschaften eines solchen Ellipsoids emulieren, ohne daß das holographische Element die äußerliche Form eines solchen Ellipsoids aufweisen muß. Des weiteren läßt sich mit Kenntnis der Form der Ellipsoid aus dem Einfallswinkel des Lichtes auf den Detektorpunkt den "Ausfallswinkel" des Lichtes aus dem Auge bzw. aus der Projektionsrichtung der Einfallswinkel auf das Auge ermitteln.
- Vorzugsweise weist das holographische Element lichtbrechende Eigenschaften bei einer oder einigen diskreten Wellenlängen auf, die einer Brechung an der Konkavseite einer gemäß der Krümmung eines rotationssymmetrischen Ellipsoids ausgebildeten Fläche entsprechen, welche Brechung einer Spiegelung an einer jeweiligen, um die Rotationsachse des Ellipsoids rotationssymmetrischen Kegelfläche entspricht, die am Ort der Brechung senkrecht zum Ellipsoid steht.

Wird das Auge als idealisiertes optisches System betrachtet, bei dem einfallendes Licht stets durch einen gemeinsamen optischen Mittelpunkt verläuft, und wird der Eingang einer Abtastvorrichtung bzw. einer daran gekoppelten Lichtleitvorrichtung als Punktdetektor betrachtet, so verlaufen auf den einen Punkt gerichteten Strahlen stets durch den anderen Punkt, wenn diese Punkte den jeweiligen Brennpunkten eines wie oben beschriebenen rotationssymmetrischen Ellipsoids eines derart realisierten holographischen Elements entsprechen. Mit Kenntnis der Form der Ellipsoid läßt sich aus dem Einfallswinkel des Lichtes auf den Detektorpunkt den "Einfallswinkel" des Lichtes auf das Auge ermitteln.

Während ein solches holographisches Element durch die holographische Aufnahme eines reellen Gegenstands schwer zu verwirklichen wäre, läßt sich ein solches holographisches Element per computergesteuerte Belichtung realisieren. Das Ergebnis ist ein leicht reproduzierbares optisches Element, das in der Lage ist, auf ein Auge gerichtete Lichtstrahlen unter Beibehaltung einer eindeutigen Zuordnung zum Einfallswinkel auf einen gemeinsamen Punkt zu brechen. Zudem lassen sich mit einem holographischen Element die angegebenen Brechungseigenschaften ohne Auswirkungen auf die äußerliche Form des holographischen Elements emulieren.

Ein besonderer Vorteil einer wie hier beschriebenen Ausgestaltung des holographischen Elements (zwecks der hiesigen Erklärung: "HE1") liegt in ihrer möglichen Kombination mit einem holographischen Element ("HE2") gemäß dem vorhergehenden Abschnitt. Sind die jeweiligen Ellipsoide gleichförmig und virtuell ortsgleich, und wird HE1 zur Brechung von direkt aus der Umgebung fallenden Lichtstrahlen ("L1-Strahlen"), während HE2 zur Brechung von Lichtstrahlen ("L2-Strahlen") zwischen dem Auge und den projizierenden bzw. lichterfassenden Komponenten des Informationssystems, so sind diejenigen auf HE1 gerichtete L1-Strahlen und diejenigen auf/von HE2 gerichteten L2-Strahlen koaxial die zwischen HE1 bzw. HE2 und der jeweiligen Projektions-, Abtast- bzw. Lichtleitvorrichtung den gleichen Strahlengang aufweisen. Dieser Vorteil wird unten unter Bezugnahme auf die Zeichnungen näher erläutert.

Im folgenden werden einzelne Merkmale der Erfindung besprochen. Zwecks der Übersichtlichkeit ist die Besprechung der Merkmale nach Themengebieten gegliedert.

### 1 Betriebsmodi

Eingangs wurde das erfindungsgemäße Zurverfügungstellen von Information" pauschal als jegliches Zurverfügungstellen von Informationen definiert, das ein augenbezogenes Zurverfügungstellen von Informationen und/oder ein Zurverfügungstellen von augenbezüglich gewonnenen Informationen umfaßt. Hierzu gehören insbesondere die Projektion von optischen Informationen auf das Auge sowie die optische Bestimmung der Orientierung des Auges.

Viele Ausführungsformen des erfindungsgemäßen Informationssystem bedürfen zum Beispiel, ein zumindest partielles Bild des dem Auge zugeordneten Gesichtfeldes und/oder ein zumindest partielles Bild ausgewählter Augenstrukturen, beispielsweise der trisfärbung oder der Netzhautgefäßstruktur, um Informationen in der gewollten Art und Weise zur Verfügung zu stellen. Bei vielen Ausführungsformen des erfindungsgemäßen Informationssystems werden mehrere Quellen informationstragender und/oder informationsliefernder optischer Signale verwendet, um beispielsweise eine Korrelation zwischen verschiedenen Informationen herzustellen oder um redundante Informationen zu gewinnen. Solche Bilder und/oder andere für das Zurverfügungstellen von Informationen wertvollen optischen Signale lassen sich beispielsweise auf folgender Art gewinnen bzw. generieren.

Da das erfindungsgemäße Informationssystem Licht über ein holographisches Element projiziert und/oder erfaßt, wird ein vorzugsweise vor dem Auge angeordnetes holographisches Element als Lichtleitvorrichtung in diesem Abschnitt ("Betriebsmodi") der Beschreibung vorausgesetzt. Dies ist jedoch nicht als Einschränkung zu verstehen. Sofern die beschriebene Abtastung bzw. Projektion im sekundären Sinne der Erfindung erfolgt, kann diese sekundäre Abtastung bzw. Projektion ohne holographisches Element oder gar ohne Lichtleitvorrichtung realisiert werden.

### 1.1 Erfassung von Licht aus dem Auge

Die Erfindung sieht drei bevorzugte Vorgehensweisen zur Gewinnung von Lichtsignalen aus dem Auge vor: die passive punktuelle Abtastung, die Abtastung bei aktiver punktueller Beleuchtung und die punktuelle Abtastung bei aktiver flächenhafter Beleuchtung. Andere Arten der Abtastung, die als nicht bevorzugt angesehen werden, werden am Ende dieses Abschnitts besprochen.

Über Vorrichtungen zur Lichtabtastung wurde eingangs ausführlich gesprochen. Bei den nachfolgend beschriebenen Vorgehensweisen zur Gewinnung von Lichtsignalen aus dem Auge wird der erfaßte Lichtstrahl vorzugsweise mittels eines holographischen Element gebrochen, das vorzugsweise vor dem Auge angeordnet ist.

### 1.1.1passive punktuelle Abtastung

Bei der passiven punktuellen Abtastung von Licht aus dem Auge dient das Umgebungslicht als Lichtquelle. Diese Art der Abtastung ist somit geeignet, Netzhaut- oder Korneareflexbilder der Umgebung oder auch die Färbung der Iris zu erfassen. Als Umgebungslicht ist hier jedoch auch das Licht zu verstehen, das zum Beispiel die körpereigenen Strukturen abstrahlen. Hierzu gehört beispielsweise das von der Netzhaut- oder Lederhautgefäßstruktur abgestrahlte Infrarotlicht, Somit können auch diese Strukturen mit dieser Art der Abtastung erfaßt werden.

Eine punktuelle, sozusagen pixelweise, Abtastung des Auge erfolgt erfindungsgemäß seriell. Dabei werden jeweilige ausgewählte Gebiete des Auges nacheinander gezielt abgetastet. Dies erfolgt vorzugsweise mit Hilfe einer entsprechenden Lichtleitvorrichtung, beispielsweise durch entsprechende Fokussierung und/oder entsprechende räumliche Einschränkung des erfaßten Lichtstrahls mittels einer entsprechenden Fokussier-, Blend-, Scanner-und/oder anderen herkömmlichen Lichtleitvorrichtungen, wie dem Fachmann für Abtastvorrichtungen bekannt ist.

Eine weitere Selektion des abgetasteten Gebiets bzw. Lichts kann zum Beispiel über eine zeitliche, farbliche oder sonstige Einschränkung des erfaßten Lichts erfolgen.

Bei geeigneter Auslegung der optischen Abtastvorrichtung und gegebenenfalls der Lichtleitvorrichtung hat die punktuelle Abtastung den Vorteil, daß die erfaßten Lichtstrahlen bei ihrem Auftreffen auf das holographische Element einen geringen Durchmesser aufweisen und somit in Falle einer unebenen virtuellen Form des holographischen Elements keine nennenswerten Verzerrungen erleiden, wohingegen bei breiten Lichtstrahlen unterschiedliche Teile des Lichtstrahls vom holographischen Element sehr unterschiedlich gebrochen werden können. (Bezüglich des Unterschieds zwischen der Form des holgraphischen Elements und der virtuellen Form des holographischen Elements wird auf die obige Beschreibung sowie auf den Abschnitt 2 "holographisches Element" verwiesen.)

Nachteilig bei der passiven Abtastung ist die Abhängigkeit des erfaßbaren Lichts an den Umgebungsumständen, d.h. an das verfügbare Umgebungslicht. Dies stellt vor allem bei der Erfassung von sichtbarem Licht aus der Umgebung bei Nacht oder Dämmerung ein Problem dar. Des weiteren kann es zu Schwierigkeiten bei der Abtastung eines Netzhautreflexbildes, da die Netzhaut lediglich ca. 4% bis 10% des darauffallenden Lichtes reflektiert.

An die Eigenschaften des holographischen Elements fordert die passive punktuelle Abtastung von Licht aus dem Auge, daß das holographische Element in der Lage ist, diejenigen aus dem Auge gerichteten Lichtstrahlen, die erfaßt werden sollen, in Richtung Abtast- bzw. Lichtleitvorrichtung zu brechen. Dabei ist zu berücksichtigen, daß Hologramme, wie oben besprochen, üblicherweise ihre Brechungswirkung lediglich bei wenigen vorbestimmten relativ schmalen Spektralbereichen aufweisen. Dies ist bei der Erfassung von beispielsweise Netzhaut- oder Korneareflexbildern der Umgebung von Nachteil, die typischerweise sehr viele Spektralkomponenten umfassen, Einige bevorzugte Ausführungen der Erfindung sehen deshalb vor, daß das holographische Element Licht bei einigen, beispielsweise 5, 10 oder gar 20, diskreten Wellenlängen bricht.

Bei der passiven Erfassung von monochromen Bildern, beispielsweise Infrarotbilder der Netzhautgefäßstruktur, ist die oben genannte Eigenschaft von holographischen Elementen unproblematisch, da das holographische Element dafür ausgelegt werden kann, Licht der relevanten Wellenlänge zu brechen.

Bei der passiven punktuellen Abtastung von Licht aus dem Auge wird vorzugsweise ein holographisches Element verwendet, das lichtbrechende Eigenschaften bei einer oder einigen diskreten Wellenlängen aufweist, die einer Spiegelung an der Konkavseite einer gemäß der Krümmung eines rotationssymmetrischen Ellipsoids ausgebildeten Fläche entsprechen.

### 1.1.2Abtastung bei aktiver punktueller Beleuchtung

Bei dieser vorteilhafte Art der Abtastung werden die zu untersuchenden Gebiete des Auges punktuell und nacheinander von einer Projektionsvorrichtung aktiv beleuchtet, während das vom jeweiligen Gebiet reflektierte Licht von der Abtastvorrichtung erfaßt wird. (Wie im Abschnitt 1.5 beschrieben wird, kann auch das vom jeweiligen Gebiet des Auges ausgestrahlte Licht gleichzeitig von der bzw. einer Abtastvorrichtung erfaßt werden.)

Auch hier werden die jeweiligen Gebiete des Auges, wie oben besprochen, vorzugsweise mit Hilfe einer entsprechenden Lichtleitvorrichtung nacheinander gezielt beleuchtet bzw. abgetastet. Interessant ist jedoch, daß bei entsprechend starker Beleuchtung im Verhältnis zum zurverfügungstehenden Umgebungslicht im oben genannten Sinne auf eine weitgehende räumliche Einschränkung des erfaßten Lichts verzichtet werden kann, ohne auf eine quasi-punktuelle Abtastung zu verzichten. Dies liegt daran, daß die Abtastvorrichtung in einem solchen Fall vorwiegend Licht erfaßt, das von dem "anvisierte" Gebiet des Auges zurückreflektiert worden ist, was einer punktuelle Abtastung entspricht.

Eine Unterscheidung des projizierten und zurückreflektierten Lichts vom Umgebungslicht läßt sich auch beispielsweise über eine Modulation erzielen. Dies könnte zum Beispiel eine Amplitudenmodulation umfassen, bei der die Intensität des projizierten Lichts mehrfach während der Projektion eines jeweiligen Pixels geändert wird, so daß der "festen" Anteil des Umgebungslichts am erfaßten Licht sich aus der aufgrund der Modulation auftretenden relativen Änderungen des erfaßten Lichts abschätzen läßt. Ähnlich könnte das projizierte Licht durch zeitliche Änderung seiner Polarisation, seiner Wellenlänge oder eines anderen seiner Kenngröße derart gekennzeichnet werden, daß es sich vom Umgebungslicht unterscheiden läßt. Viele hierfür in Frage kommende Signaltrennungsverfahren sind dem Signalverarbeitungsfachmann bekannt.

Eine wie oben beschriebene Konstellation des erfindungsgemäßen Informationssystem, bei der auf eine weitgehende räumliche Einschränkung des erfaßten Lichts verzichtet wird, könnte zu einer Vereinfachung der Abtastvorrichtung bzw. der Lichtleitvorrichtung führen und wäre beispielsweise bei einem Eyetracker anwendbar, bei dem unwahrnehmbares Infrarotlicht mit unschädlicher, jedoch im Verhältnis zum Umgebungslicht starker Intensität zur Verfolgung des Augenbewegungen in das Auge gestrahlt wird.

Interessant ist auch, daß im Falle einer Abtastung der Netzhaut unter Verwendung aktiver punktueller Beleuchtung die Iris als Blende fungiert, die vorwiegend das Licht aus dem Auge strahlen läßt, das näherungsweise parallel zum Beleuchtungsstrahl von der Netzhaut zurückreflektiert worden ist. Aus diesem Grund wird bei dieser Art der Abtastung eine konfokale Anordnung der Abtastvorrichtung und der Projektionsvorrichtung bevorzugt.

Auch bei einer Abtastung bei aktiver punktueller Beleuchtung ergibt sich aufgrund des schmalen, punktgerichteten Lichtstrahls der Vorteil der geringen Verzerrung des Lichtstrahls durch das holographische Element. Eine solche Abtastung leidet jedoch nicht an den Nachteilen einer passiven Abtastung.

Bezüglich der Realisierung des holographischen Elements bietet die Abtastung bei aktiver punktueller Beleuchtung erhebliche Vorteile. Dies liegt vor allem daran, daß die Wellenlänge des von der Projektionsvorrichtung ausgestrahlten Lichts an die Lichtbrechungseigenschaften des holographischen Elements angepaßt werden kann, oder umgekehrt. Da der zurückreflektierte Anteil des von der Projektionsvorrichtung projizierten Lichts die Grundlage der Abtastung bildet, wäre das holographische Element somit ebenfalls an die Wellenlänge des zu erfassenden Lichts angepaßt.

Bei der Abtastung bei aktiver punktueller Beleuchtung wird vorzugsweise ein holographisches Element verwendet, das lichtbrechende Eigenschaften bei einer oder einigen diskreten Wellenlängen aufweist, die einer Spiegelung an der Konkavseite einer gemäß der Krümmung eines rotationssymmetrischen Ellipsoids ausgebildeten Fläche entsprechen.

### 1.1.3punktuelle Abtastung bei aktiver flächenhafter Beleuchtung

Bei dieser Art der Abtastung werden die zu untersuchenden Gebiete des Auges flächenhaft von einer Projektionsvorrichtung aktiv beleuchtet, während jeweilig ausgewählte punktuelle Gebiete des Auges, wie oben besprochen, vorzugsweise mit Hilfe einer entsprechenden Lichtleitvorrichtung nacheinander gezielt abgetastet werden. Somit kann beispielsweise eine Abtastung okularer Strukturen auch dann erfolgen, wenn kein ausreichendes Umgebungslicht für eine solche Abtastung vorhanden ist. Allerdings ist eine Abtastung eines okularen Reflexbildes der Umgebung nicht möglich, da das erfaßte Licht nicht aus der Umgebung stammt.

Aufgrund der aktiven Beleuchtung läßt sich, wie im vorherigen Abschnitt beschrieben wurde, auch hier eine Unterscheidung des projizierten und zurückreflektierten Lichts vom Umgebungslicht beispielsweise über eine Modulation erzielen.

Ebenfalls bietet sich hier der Vorteil einer Anpassung der Wellenlänge des projizierten bzw. erfaßten Lichtes an die Lichtbrechungseigenschaften des holographischen Elements, wobei die Beleuchtung bzw. Projektion und/oder die Abtastung über das holographische Element erfolgen kann.

Nachteilig bei dieser Art der Abtastung sind die hohen Anforderungen an die Abtastvorrichtung und die ihr eventuell zugeordneten Lichtleitvorichtungen, um eine gezielte Abtastung durchzuführen. Allerdings profitiert ein derart ausgebildetes Informationssystem von der Tatsache, daß das holographische Element keine nennenswerten Verzerrungen bei einem schmalen, punktgerichteten Abtaststrahl verursacht, wie dies bei einem breiten Strahl der Fall wäre.

Bei der punktuellen Abtastung bei aktiver flächenhafter Beleuchtung wird vorzugsweise ein holographisches Element verwendet, das lichtbrechende Eigenschaften bei einer oder einigen diskreten Wellenlängen aufweist, die einer Spiegelung an der Konkavseite einer gemäß der Krümmung eines rotationssymmetrischen Ellipsoids ausgebildeten Fläche entsprechen.

### 1.1.4andere Abtastarten

Selbstverständlich besteht auch die Möglichkeit einer flächenhaften, sprich parallelen anstatt seriellen, Abtastung. Die Vorgehensweise einer solchen Abtastung ist wohl bekannt und bedarf an dieser Stelle keine nähere Erläuterung.

Es sei jedoch darauf hingewiesen, daß die flächenhafte Abtastung in Zusammenhang mit der von der Erfindung bevorzugten Form einer kompakten Brille bei kostengünstiger Ausführung üblicherweise zu unerwünschten optischen Verzerrungen führt, weshalb diese Ausführungsform als zweitrangig betrachtet wird.

### 1.2 konfokale Erfassung von Licht quasi-direkt aus der Umgebung

Das erfindungsgemäße Informationssystem erfaßt auf das Auge fallende Licht mittels einer optischen Abtastvorrichtung. Wie oben besprochen muß dieses Licht erfindungsgemäß das Auge nicht tatsächlich treffen, sondern kann auch einfach auf das Auge gerichtet gewesen sein, bevor es vom Informationssystem erfaßt bzw. zur Erfassung gelenkt wird.

Ein besonders vorteilhafte Ausführung des erfindungsgemäßen Informationssystem weist eine Vorrichtung auf, die eine zum Auge konfokale Erfassung von Licht direkt oder quasi-direkt aus der Umgebung ermöglicht. Auf diese Art und Weise läßt sich beispielsweise ein Bild des wahrgenommenen Gesichtsfeldes gewinnen, das frei von Zerzerrungen durch das optische System des Auges ist. Verschiedene solche Vorrichtungen sind sowohl dem Fachmann ohne weiteres vorstellbar als auch in den Anmeldungen PCT/EP00/09840. PCT/EP00/09841 und PCT/EP00/09843 detailliert beschrieben, weshalb auf eine Wiederholung jener Angaben hier verzichtet wird.

Erfindungsgemäß kann allerdings eine vorzugsweise zum Auge konfokale Erfassung von Licht quasi-direkt aus der Umgebung. d.h. ohne Umleitung über das Auge, über das holographische Element erfolgen. Dieses wird vorzugsweise vor dem Auge angeordnet. Ein besonderer Vorteil der Verwendung eines holographischen Element zur Lichtbrechung liegt sowohl darin, daß seine Brechungseigenschaften, wie oben beschrieben, nicht ausschließlich durch seine äußere Form bestimmt sind, als auch darin, daß sich seine Brechung präzis, leicht reproduzierbar, wellenlängenselektiv und/oder winkelselektiv gestalten läßt.

Gemäß einer bevorzugten Ausführung der Erfindung weist das holographische Element hierzu die oben beschriebenen lichtbrechenden Eigenschaften bei einer oder einigen diskreten Wellenlängen auf, die einer Brechung an der Konkavseite einer gemäß der Krümmung eines rotationssymmetrischen Ellipsoids ausgebildeten Fläche entsprechen, welche Brechung einer Spiegelung an einer jeweiligen, um die Rotationsachse des Ellipsoids rotationssymmetrischen Kegelfläche entspricht, die am Ort der Brechung senkrecht zum Ellipsoid steht.

Bei einer solchen Ausführung muß der Eingang der Abtastvorrichtung bzw. einer daran gekoppelten Lichtleitvorrichtung an einem der Brennpunkte des Ellipsoids liegen und dem holographischen Element zugewandt sein, um das vom holographischen Element gebrochene Umgebungslicht zu erfassen. Werden zudem Maßnahmen ergriffen, die dafür sorgen, daß der andere Brennpunkt des Ellipsoids ungefähr mit dem optischen Mittelpunkt des optischen Systems des Auges übereinstimmt, so werden diejenigen Lichtstrahlen erfaßt, die konfokale zum optischen System des Auges aus der Umgebung auf das holographische Element fallen. Solche Maßnahmen können beispielsweise die Möglichkeit umfassen, die Position des holographischen Elements gegebenenfalls samt optischer Abtastvorrichtung und/oder Lichtleitvorrichtung mechanisch oder manuell zu verstellen.

### 1.3 Projektion

Wie oben besprochen, kann eine Projektion erfindungsgemäß sowohl bei der Gewinnung von Informationen aus dem Auge als auch beim Zurverfügungstellen von Informationen zum Tragen kommen. Über Vorrichtungen zur Lichtprojektion wurde eingangs ausführlich gesprochen.

Bei der Projektion wird vorzugsweise ein schmaler Lichtstrahl und/oder ein holographisches Element verwendet, das lichtbrechende Eigenschaften bei einer oder einigen diskreten Wellenlängen aufweist, die vorzugsweise einer Spiegelung an der Konkavseite einer gemäß der Krümmung eines rotationssymmetrischen Ellipsoids ausgebildeten Fläche entsprechen, und/oder das vorzugsweise vor dem Auge angeordnet ist. Die vorteile sowie weitere vorteilhafte Ausgestaltungsmöglichkeiten eines solchen holographischen Elements wurden oben beschrieben.

### 1.3.1 Projektion zur Informationsgewinnung

Bei der Projektion zu Informationsgewinnung wird Licht von einer Projektionsvorrichtung derart projiziert, daß es reflektiert und zwecks einer Informationsgewinnung erfaßt werden kann. Zur Gewinnung von augenbezüglichen Informationen wird das Licht auf das Auge projiziert und von diesem reflektiert.

Zwei wesentliche Ausführungsarten einer solchen Projektion wurden oben unter den Überschriften "Abtastung bei aktiver punktueller Beleuchtung" und "punktuelle Abtastung bei aktiver flächenhafter Beleuchtung" beschrieben. Ebenfalls könnte das Auge aktiv flächenhaft beleuchtet und flächenhaft abgetastet werden. Je nach Ausführung sind diese Projektionsarten im primären oder sekundären Sinne der Erfindung anwendbar.

Im sekundären Sinne der Erfindung wären andere Projektionsarten in Verbindung mit entsprechenden Abtastarten sinnvoll einsetzbar. Beispielsweise könnte Licht auf ein Teil des Informationssystem oder in die Umgebung gemäß einer der beschriebenen Projektions- bzw. Abtastverfahren projiziert, dort reflektiert und schließlich vom Informationssystem erfaßt werden. Eine solche Informationsgewinnung könnte beispielsweise dazu dienen, die optischen Komponenten des Informationssystems zu eichen oder Information aus der Umgebung zu gewinnen.

Bei der Projektion zur Informationsgewinnung wird das projizierte Licht vorzugsweise moduliert oder auf sonstige Weise gekennzeichnet, um beispielsweise dieses Licht vom Umgebungslicht unterscheiden zu können. Eine Modulation kann beispielsweise auch dazu dienen, die Laufzeit des Lichtes zwischen Projektionsvorrichtung und Abtastvorrichtung bestimmen zu können.

Des weiteren wird bei der Projektion zu Informationsgewinnung vorzugsweise Infrarotlicht projiziert, um das vom Mensch wahrgenommene Szene nicht zu beeinträchtigen.

### 1.3.2 Projektion zum Zurverfügungstellen von Informationen

Grundsätzlich entspricht jede Projektion von Licht einer Übertragung, das heißt einem Zurverfügungstellen, von Informationen.

Im primären Sinne der Erfindung wird Information dadurch mittels einer Projektion zur Verfügung gestellt, daß optische Informationen in Form von Licht über ein holographisches Element in das Auge projiziert werden. Damit die Informationen vom Auge wahrnehmbar sind, wird das Licht auf die Netzhaut projiziert. Damit das Licht die gewünschten optischen Informationen übertragen kann, wird das Licht einer entsprechenden Modulation unterworfen. Beispielsweise wird Licht bei einer roten, einer blauen und einen grünen Wellenlänge derart pixelweise intensitätsmoduliert projiziert, daß das Licht aufgrund des wohlbekannten Prinzips der Farbaddition als vollfarbiges Bild wahrgenommen wird.

Gemäß der logischen Schlußfolgerung der beiden vorhergehenden Absätzen umfaßt eine Projektion zum Zurverfügungstellen von Informationen im sekundären Sinne der Erfindung jede andere oder weitere Projektion von Licht. Beispielsweise kann das erfindungsgemäße Informationssystem Licht projizieren, das später vom Informationssystem selbst abgetastet und zum Zwecke einer Eichung oder einer Justierung ausgewertet wird: Ebenfalls könnte Licht in die Umgebung projiziert werden, um dort von einem Menschen direkt oder indirekt wahrgenommen oder von einem anderen System erfaßt zu werden.

Die Verwendung eines holographisches Elements als Lichtleitvorrichtung bei der Projektion bietet unter anderem den Vorteil, daß sich die Brechungseigenschaften des holographischen Elements an die Wellenlänge an die Wellenlänge des projizierten Lichts angepaßt werden können, oder ungekehrt. Dies könnte zum Beispiel dazu dienen, sichtbares Licht von einer Projektionsvorrichtung in das Auge zu leiten, während Infrarotlicht aus der selben Projektionsvorrichtung zwecks Steuerung eines anderen Systems in die Umgebung geleitet wird.

Während sowohl die serielle als auch die parallele Projektion von Licht für das Zurverfügungstellen von Informationen angewandt werden kann, wird die serielle, pixelweise Projektion bevorzugt. Dies liegt insbesondere daran, daß die serielle Projektion die Verwendung eines sehr schmalen Lichtstrahls ermöglicht, dessen Strahlenform auch bei einer Brechung an einer gekrümmten oder virtuell gekrümmten Fläche im wesentlichen unverändert bleibt. (Bezüglich der Deutung des Begriffs "virtuell' gekrümmt" wird auf den Abschitt 2, "Hologramm" verwiesen.)

### 1.4 Erfassung der Umgebung über Sensorik

Vorzugsweise weist das erfindungsgemäße Informationssystem eine Sensorik auf, die bevorzugt Informationen bezüglich der Umgebung liefert.

Wie in den Anmeldungen PCT/EP00/09840, PCT/CP00/09841 und PCT/EP00/09843 detailliert beschrieben wurde, kann es sehr vorteilhaft sein, wenn das erfindungsgemäße Informationssystem über Sensorik zur Erfassung von Umgebungsdaten verfügt. Solche Umgebungsdaten können zum Beispiel dazu verwendet werden, um die Lage und Orientierung des Informationssystem und/oder des Auges gegenüber der Umgebung zu bestimmen. Ebenfalls können Umgebungsdaten dazu verwendet werden, um Informationen in Korrelation mit dem auf das Auge fallenden Licht zur Verfügung zu stellen.

Nachfolgend soll auf zwei dieser Sensorsysteme kurz eingegangen werden, die in besonders vorteilhafter zum Zurverfügungstellen von Informationen in Korrelation mit auf ein Auge fallendem Licht angewandt werden können.

### 1.4.1 Kamera

Vorzugsweise weist das erfindungsgemäße Informationssystem eine Kamera auf.

Die Erfassung von Licht aus der Umgebung über eine Kamera, beispielsweise eine CCD-Kamera, bietet eine kostengünstige Möglichkeit, hochwertige optische Signale aus der Umgebung zu erfassen. Vorzugsweise soll die optische Aufnahmeachse der Kamera so nah wie möglich mit der optischen Achse des Auges in seiner neutralen, das heißt geradeaus schauenden, Stellung übereinstimmen, um ein mit dem Auge nahezu konfokales und somit im vergleich zum wahrgenommenen Bild im wesentlichen parallaxe-freies Bild zu liefern.

Wird das von der Kamera erfaßt Bild teilweise oder insgesamt mit aus dem Auge gewonnenen Reflexbildern der Umgebung verglichen, läßt sich beispielsweise aus dem Verhältnis der Reflexbilder zu gleichzeitig erkannten Strukturen des Auges der vom Auge "anvisierte" Bereich der Umgebung präzis bestimmen.

### 1.4.2 Lagesensorik

Vorzugsweise weist das erfindungsgemäße Informationssystem eine Lagesensorik auf.

Über Kenntnis der optischen Eigenschaften der Komponenten des erfindungsgemäßen Informationssystems läßt sich in der Regel den Strahlengang eines abgetasteten Lichtstrahls präzis bestimmen. Durch entsprechende Auswertung vom Auge gewonnenen Informationen läßt sich somit auch die Orientierung des Auges gegenüber der Komponenten des Informationssystems präzis bestimmen.

Durch eine im Informationssystem eingebundene Lagesensorik, beispielsweise eine IR- oder RF-Triangulationsvorrichtung, ein GPS-Empfänger und/oder Gyrosensoren, die gegebenenfalls mit fernen Komponenten, beispielsweise festpositionierte Sender, Satelliten oder ähnliches, kooperiert, läßt sich die Lage und/oder Orientierung des Informationssystems ermitteln. Somit kann auch die Lage und/oder Orientierung des Auges bezüglich der Umgebung bestimmt werden. Selbstverständlich kann eine solche Lagesensorik auch anderen im Rahmen eines Zurverfügungstellens oder einer Gewinnung von Informationen liegenden Zwecken dienen.

### 1.5 Mischbetrieb

Wie eingangs erwähnt wurde, lassen sich die oben beschriebenen Verfahren zum Zurverfügungstellen oder zur Gewinnung von Informationen beliebig kombinieren.

Eine besonders vorteilhafte Ausführung des erfindungsgemäßen Informationssystems erfaßt optische Signale aus der Umgebung sowohl über ein bei 1-5 Wellenlängen lichtbrechendes ellipsoidartiges holographisches Element gemäß Abschnitt 1.2 als auch über eine Kamera gemäß Abschnitt 1.4.1. Zudem erfaßt es mittels optischer Signale im Infrarotbereich gemäß Abschnitt 1.1.2 über ein ebenfalls ellipsoidartiges holographisches Element Informationen über die Netzhautstruktur. Durch gleiche Gestaltung und Lage der Ellipsoide sowie einen zwischen holographisches Element und Abtastvorrichtung gleichen Strahlengang wird erreicht, daß ein wie eingangs beschriebener vorteilhafter koaxialer Zusammenhang zwischen den aus dem Auge und den aus der Umgebung über die Abtastvorrichtung erfaßten Lichtstrahlen besteht. Mittels einer geeigneten Mustervergleich der redundant erfaßten Informationen aus der Umgebung läßt sich auch zwischen den jeweilig aus der Umgebung erfaßten Informationen einen präzisen Zusammenhang ermitteln. Somit läßt sich beispielsweise die Blickrichtung des Auges gegenüber der Umgebung auf einfache Art präzis bestimmen.

Vorteilhaft an der geschilderten Ausführung ist,
- daß das holographische Element wenige (im Grenzfall zwei) holographischen Aufnahmen umfassen muß,
- daß keine optisch hochwertige Abtastvorrichtung notwendig ist, da die räumliche Beschränkung abgetasteten Bereichs über die Projektion erfolgen kann, und
- daß die zur Informationsgewinnung eingesetzte Projektion im nichtwahrnehmbaren Infrarotbereich erfolgen kann.

Das oben beschriebene Beispiel schildert lediglich eine der unzähligen Kombinationsmöglichkeiten und dient somit im wesentlichen zur Exemplifizierung der möglichen Vorteile eines im "Mischbetrieb" ausgeführten Informationssystems.

Insbesondere sei darauf hinzuweisen, daß die Kombinationen einer Projektion und/oder Erfassung sichtbaren Lichts und einer Projektion und/oder Erfassung nichtwahrnehmbares Infrarotlicht zu besonders vorteilhaften Ausgestaltungen der Erfindung führen kann. Dabei können auch mehrere Wellenlängen des jeweiligen Lichtarts zum Einsatz kommen. Wie zuvor erwähnt, bietet sich im Falle einer Projektion und/oder Erfassung über ein holographisches Element eine Anpassung der lichtbrechenden Eigenschaften des holographischen Elements an die Wellenlängen des zu projizierenden bzw. zu erfassenden Lichts an.

### 1.6 Flying-Spot

Abtast- und Projektionsverfahren, bei dem räumlich begrenzte Gebiete typischerweise entlang einer geraden oder gekrümmten Linie seriell abgetastet bzw. beleuchtet werden, werden in der Fachsprache häufig als "Flying-Spot"-Verfahren (zu deutsch: " fliegender Punkt"-Verfahren) bezeichnet.

Bei vielen herkömmlichen augenbezogenen Informationssystemen werden optische Signals mittels eines flächigen Detektors flächenartig aus dem Auge erfaßt bzw. mittels eines flächigen Projektors flächenartig in das Auge projiziert. Diese Vorgehensweise birgt den Nachteil, daß eine optisch korrekte Abbildung eines gekrümmten Augenteils auf einen flächigen Detektor bzw. eines flächigen Projektors auf ein gekrümmtes Augenteil nur mit erheblichem Aufwand zu erzielen ist.

Dieses Problem tritt beim Flying-Spot-Verfahren nur im erheblich verringertem Maße auf.

Das Flying-Spot-Verfahren wird zudem aufgrund seiner Kompatibilität mit einem holographischen Element vorzugsweise beim erfindungsgemäßen Informationssystem angewandt.

### 1.6.1 Spiral-, Kreis- oder Ellipsenscan

Die menschliche monookulare Wahrnehmung ist im wesentlichen rotationssymmetrisch um eine durch die Fovea centralis und dem optischen Mittelpunkt der Linse verlaufende Sehachse. Dementsprechend sind viele Teile des Auges, zum Beispiel die Iris, die Pupille, die Kornea, die Linse und in manchen Hinsichten auch die Retina, bei den meisten Menschen näherungsweise rotationssymmetrisch um die Sehachse ausgebildet.

Das Auge wird deshalb erfindungsgemäß vorzugsweise gemäß dem Flying-Spot-Verfahren mit einem spiral- oder kreisförmigen Abtast- bzw. Projektionsmuster, vorzugsweise um die Sehachse, überstreichen, wobei unter "kreisförmig" eine Vielzahl von konzentrischen Kreisen verstanden werden kann. Stehen die Projektions- bzw. Abtaststrahlen entsprechend schräg zur Sehachse, kann die Verwendung eines ellipsenförmigen Abtast- bzw. Projektionsmusters vorteilhaft sein, wie in der DE 197 28 890 A1 beschrieben ist.

Es sei betont, daß der Begriff Abtast- bzw. Projektionsmuster hier als das quasi zweidimensionale Bewegungsmuster zu verstehen ist, das der Ausgangs- bzw. Endpunkt des Strahlengangs des von der Abtastvorrichtung aus dem Auge erfaßten Lichtes bzw. des vom Projektionssystem in das Auge projizierten Lichtes im Auge beschreibt.

### 1.6.2 Strahlen senkrecht zum Auge

Fallen Lichtstrahlen senkrecht auf den Luft-Augapfel-Übergang, so wird ein gewisser Anteil des Lichtes in entgegengesetzte Richtung zum einfallenden Lichtstrahl zurückreflektiert, während der restliche Anteil quasi ungehindert durchstrahlt, wonach es von "tieferliegenden". Teilen des Auges absorbiert bzw. gestreut wird. Ersteres gilt analog für über den Kornea-Luft-Übergang aus dem Auge austretende Lichtstrahlen.

Beim erfindungsgemäßen Informationssystem wird vorzugsweise gemäß dem Flying-Spot-Verfahren projiziert bzw. abgetastet. Dabei wird vorzugsweise ein "schmaler" Lichtstrahl verwendet, der am Luft-Augapfel-Übergang einen im Vergleich zur Augapfelkrümmung, insbesondere zur Korneakrümmung, unwesentlichen Durchmesser aufweist. Ebenfalls kann ein Lichtstrahl mit einem "schmalen" Durchmesser gemäß Abschnitt 3.1 "Strahldurchmesser" verwendet werden. Der Lichtstrahl wird vorzugsweise derart projiziert bzw. abgetastet, daß alle seiner Einzelstrahlen den Luft-Augapfel-Übergang möglichst senkrecht begegnen.

Die Kornea, das heißt der Luft-Kornea-Übergang, verursacht ungefähr 80% der vom Auge auf einen darauffallenden Lichtstrahl ausgeübten Brechung. Somit hat die- oben beschriebene Vorgehensweise nicht nur den Vorteil, daß wenig Licht am Luft-Kornea-Übergang in eine unnützliche Richtung gebrochen wird, sondern auch den Vorteil, daß die Strahlen eine geringe Brechung durch das optische System des Auges erfahren. Dies wirkt sich nicht nur auf die räumliche Projektions- bzw. Abtastgenauigkeit positiv aus, sondern ist auch bei Anwendungen vorteilhaft, bei denen die Geometrie der Lichtstrahlen eine wesentliche Rolle spielt. Dies ist beispielsweise bei Eyetracker-Anwendungen der Fall.

Die Tatsache, daß senkrecht auf das Auge fallende Strahlen partiell in entgegengesetzte Richtung zurückreflektiert werden, kann dazu verwendet werden, Informationen über die Topologie des Auges zu gewinnen. Dies kann zum Beispiel über eine eine Projektionsvorrichtung und eine Abtastvorrichtung umfassende Projektor-Detektor-Anordnung erfolgen, die Licht ungefähr senkrecht auf das Auge projiziert und anschließend die Koaxialität des erfaßten zurückreflektierten Lichtstrahls und des projizierten Lichtstrahls ermittelt. Sind diese Lichtstrahlen nicht im wesentlichen (insbesondere die Korneaoberfläche weist viele mikro- und makroskopische Unregelmäßigkeiten aus und darf deshalb nicht als glatt reflektierende Oberfläche betrachtet werden) koaxial, so kann daraus geschlossen werden, daß der projizierte Lichtstrahl nicht senkrecht auf das Auge traf. Solche Informationen über die Topologie des Auges können unter anderem zur Ermittlung der Lage und/oder Orientierung des Auges verwendet werden.

Für eine derartige Projektor-Detektor-Anordnung bietet sich eine konfolcale Anordnung der Projektionsvorrichtung und der Abtastvorrichtung, beispielsweise über einen Teilerspiegel. Eine in diesem Zusammenhang vorteilhafte, konzentrische Detektorbereiche aufweisende Abtastvorrichtung ist im Abschnitt 3.4 ("besondere Abtastvorrichtungen") beschrieben.

Die Verwendung eines holographischen Elements ist insbesondere bei der Projektion bzw. der Erfassung eines senkrecht zum Auge verlaufenden Lichtstrahls vorteilhaft, da sie eine einfache derartige virtuelle Ausgestaltung (vgl. Abschnitt 2 "-holographisches Element") einer komplexen Lichtleitvorrichtung ermöglicht, daß Lichtstrahlen aus mehreren oder sogar einer einzigen Projektionsvorrichtung senkrecht auf verschiedene Gebiete des Auges und/oder von verschiedenen Gebiete des Auges senkrecht austretende bzw. zurückreflektierte Lichtstrahlen in mehrere oder sogar eine einzige Abtastvorrichtung gelenkt werden können.

Ein derartiges holographisches Element läßt sich insbesondere durch computergestützte Verfahren herstellen. Dabei sind der Gestaltung kaum Grenzen gesetzt. Grundsätzlich notwendig ist lediglich, daß der Strahlengang eines senkrecht zum Auge stehenden Strahls über das holographische Element zu einer Projektionsvorrichtungen, einer Abtastvorrichtungen und/oder einer daran gekoppelten Lichtleitvorrichtung führt. Vorzugsweise wird bei der Gestaltung des holographischen Elements dafür Sorge getragen, daß der Einfall- bzw. Ausfallwinkel des Strahls an der Abtastvorrichtungen, der Projektionsvorrichtungen und/oder einer daran gekoppelten Lichtleitvorrichtung eindeutige Rückschlüsse auf ein jeweiliges dem Strahl zuzuordnendes Gebiet des Auges ermöglicht, d.h. daß der Einfall- bzw. Ausfallwinkel des Strahls an der Abtastvorrichtungen, der Projektionsvorrichtungen und/oder einer daran gekoppelten Lichtleitvorrichtung eine eindeutige Abbildung jeweiliger Gebiete des Auges darstellt, und umgekehrt.

Da das Auge nicht rund und zudem schwenkbar ist, ist eine Lichtleitvorrichtung, insbesondere ein holographisches Element, die stets in der Lage ist, Projektionsstrahlen senkrecht auf ein beliebiges freigelegtes Gebiete eines Auge zu richten (oder umgekehrt für Abtaststrahlen) kaum realisierbar. Insbesondere ist problematisch, daß das Auge typischerweise nicht um einen Punkt schwenkt, der mit dem Mittelpunkt der Krümmung der Kornea übereinstimmt. Die Lichtleitvorrichtung wird deshalb vorzugsweise gemäß einem empirischen Modell des zu erfassenden Gebietes eines Auges unter der Annahme gestaltet, daß das Auge sich gewöhnlicherweise innerhalb eines bestimmten Drehbereichs befindet. Alternativ kann die Lichtleitvorrichtung als gute Näherung gemäß einem aus empirischen Daten gewonnenen Modell eines kugelförmigen Auges gestaltet werden.

### 2 Das holographische Element

Das holographische Element spielt bei der vorliegenden Erfindung als Lichtleitvorrichtung eine entscheidende Rolle. Eingangs wurden grundlegende Eigenschaften des holographischen Elements beschrieben. Hervorgehoben wurde dort insbesondere die Fähigkeit eines holographischen Elements, die Brechungseigenschaften eines Gegenstands in gewissen Maßen unter den bestimmten Umständen der holographischen Wiedergabe nachzuahmen.

Extrem vereinfacht dargestellt, kann ein Hologramm als Aufnahme des Interferenzmusters zweier Lichtstrahlen, nämlich eines Objektstrahls und eines Referenzstrahls, angesehen werden. Fällt Licht entsprechend dem Referenzstrahl auf das fertige Hologramm, so wird es durch die Brechungscharakteristik des Interferenzmusters in einen dem Objektstrahl entsprechenden Lichtstrahl "umgewandelt". Typischerweise entspricht der Objektstrahl dem von einem reellen Gegenstand gebrochenen Licht. Dementsprechend kann durch Beleuchtung des Hologramms mit dem Referenzstrahl quasi das von einem reellen Gegenstand gebrochene Licht in Abwesenheit dieses Gegenstands sichtbar werden; der Gegenstand wird sozusagen "virtuell" sichtbar. Auf diese Weise ahmt das Hologramm die Form, das heißt die Brechungseigenschaften, des Gegenstands "virtuell"' nach.

Vorteilhaft dabei ist, daß die physikalische Form des Hologramms, die typischerweise folienartig oder als Beschichtung ausgeführt wird, keinesfalls mit der Form des aufgenommenen Gegenstands, das heißt der "virtuellen" Form des Hologramms, übereinstimmen muß. Dementsprechend ist die virtuelle Form des Hologramms gemäß den Systemerfordernissen frei wählbar. Allerdings beeinflussen Formunterschiede des holographischen Photomaterials zwischen Aufnahme und Wiedergabe das Interferenzmuster und somit auch die Wiedergabe. Dies ist gegebenenfalls entsprechend zu berücksichtigen.

Zu erwähnen seien auch elektro-holographische Elemente, die ebenfalls zur Gattung der holographischen Elemente gehören. Elektro-holographische Elemente sind holographische Elemente, deren holographische Inhalt sich durch Anlegen einer Spannung ändern kann. Bezüglich der Herstellung und der genauen Bedienung von elektro-holographischen Elementen wird auf die einschlägige Fachliteratur verwiesen.

### 2.1 Herstellungsverfahren

Das Herstellen von Hologramme ist ein sehr komplexer Vorgang, dessen genaue Beschreibung den Rahmen dieser Anmeldung sprengen würde. Es wurde deshalb eingangs einige beispielhafte Veröffentlichung zu diesem Thema namentlich erwähnt.

Das Herstellen von Hologramme läßt sich in zwei Grundkategorien unterteilen. Diese sind die computergestützte Herstellung und die holographische Aufnahme eines reellen Gegenstands. Jeweils ein typisches Beispiel dieser Herstellungsarten sollen im folgenden kurz beschrieben werden.

### 2.1.1reeller Gegenstand

Vorzugsweise weist das : erfindungsgemäße Informationssystem ein eine holographische Aufnahme eines reellen Gegenstands umfassendes holographisches Element auf.

Ein holographisches Element läßt sich am einfachsten mittels einer holographischen Aufnahme eines reellen Gegenstands herstellen. Dabei werden üblicherweise ein quasi direkt aus der Lichtquelle stammender Referenzstrahl und ein am reellen Gegenstand gebrochener Objektstrahl gleichzeitig auf das holographische Photomaterial projiziert. Da die scharfe Aufnahme eines Interferenzmusters ein "Stillhalten" des Interferenzmusters über die Dauer der Aufnahme erfordert, werden normalerweise Laserstrahlen als Objekt- und Referenzstrahl benutzt. Damit lediglich eine Laserlichtquelle benötigt wird, kann der aus der Laserlichtquelle stammende Laserstrahl über ein Teilerspiegel zweigeteilt werden; wobei einer das Photomaterial direkt und der andere das Photomaterial indirekt über den Gegenstand bestrahlt.

Auf diese weise können zeitlich unveränderliche Lichtleitvorrichtung, beispielsweise Spiegel, diffundierende Platten, Linsen und dergleichen holographisch aufgenommen werden, das heißt über ein holographisches Element emuliert werden.

### 2.1.2computergestützte Herstellung

Vorzugsweise weist das erfindungsgemäße Informationssystem ein mittels computergestützer Herstellung fabriziertes holographisches Element auf.

Ein mit der Stützung eines Computer hergestelltes holographisches Element wird typischerweise über sein Interferenzmuster definiert und hergestellt. Dabei wird typischerweise zuerst mittels einer Rendering- oder einer anderen Lichtausbreitungsberechnungssoftware beispielsweise aus einem Computermodell eines fiktiven Gegenstands oder anhand eines gewünschten Strahlengangmusters ein fiktiver Objektstrahl ermittelt. Aus dem fiktiven Objektstrahl und einem ebenfalls fiktiven, als Computerdaten definierten Referenzstrahl wird das Interferenzmuster der beiden Strahlen berechnet. Das resultierende Interferenzmuster wird üblicherweise sowohl phasenmäßig als auch amplitudenmäßig zwecks Vereinfachung der Herstellung quantisiert. Das quantisierte Interferenzmuster wird nachfolgend als quantisierte Amplitudenmaske, als mehrstufige Phasenreliefstruktur oder als Kombination dieser geplottet. Falls die resultierende Maske bzw. Struktur nicht die notwendige hohe Auflösung aufweist, wird photographisch verkleinert. Alternativ kann die Maske bzw. Struktur direkt mittels eines hochauflösenden Elektronenstrahllithographiesystems oder eines hochauflösenden Laserstrahlbelichtungssystems anstelle des Plottens und des Verkleinerns hergestellt werden.

Die in der Anmeldung erwähnten holographischen Elemente mit einer ellipsoidartigen virtuellen Form lassen sich vorzugsweise computergestützt herstellen.

Aus der vorhergehenden Beschreibung wird ersichtlich, daß der Begriff "virtueller Gegenstand " für den Fall eines computergestützt hergestellten holographisches Element einschränkend wirkt, da mannigfaltige Brechungs-, Beugungs- und Reflektionseigenschaften holographisch abbildbar sind, die keinem reellen Gegenstand entsprechen. Um diese unerwünschte Einschränkung entgegenzuwirken, wird hier explizit darauf hingewiesen, daß ein holographisches Element einen beliebigen holographischen Inhalt aufweisen darf, auch wenn dieser holographische Inhalt der Klarheit halber als "virtueller Gegenstand" bezeichnet wird.

### 2.1.3ein- und mehrfarbige Ausgestaltung

Da das in einem Hologramm festgehaltene Interferenzmuster lediglich auf diejenigen Lichtstrahlen eine Brechungswirkung ausüben, deren Wellenlänge, Phase und Einfallswinkel dem Interferenzmuster wie ein Schüssel zum Schloß passen, wirken einfache Hologramm im wesentlichen durchsichtig. Dies ist für die vorliegende Erfindung insbesondere dann von Vorteil, wenn das holographische Element vor dem Auge plaziert werden soll.

Wird ein Hologramm mit Licht einer einzigen Wellenlänge aufgenommen, so kann das Hologramm eine Brechung im wesentlichen nur auf Licht der gleichen Wellenlänge ausüben. In der Anmeldung wird dies als Brechung "bei einer diskreten Wellenlänge" bezeichnet.

Alternativ kann das erfindungsgemäße holographische Element Licht "bei einigen diskreten Wellenlänge" brechen. Dies kann auf zwei grundsätzlich verschiedene Weisen erreicht werden.

Zum einen können mit dem gleichen Photomaterial mehrere holographischen Aufnahmen bei jeweils unterschiedlichen Wellenlängen aber bei sonst gleichen Umständen nacheinander gemacht werden. Hieraus resultiert ein einziges Hologramm, das die Brechungseigenschaften eines einziges Gegenstands bei einigen diskreten Wellenlängen nachzuahmen vermag.

Zum anderen können mehrere holographischen Aufnahmen bei jeweils unterschiedlichen Wellenlängen aber bei sonst gleichen Umständen nacheinander gemacht werden, wobei das Photomaterial nach jeder Aufnahme ausgetauscht wird. Die einzelnen Aufnahmen können später schichtweise zusammengelegt werden, um ein Gesamthologramm zu erzeugen, das die Brechungseigenschaften eines einziges Gegenstands bei einigen diskreten Wellenlängen nachahmen kann. Allerdings ist es schwierig, das Register beim Zusammenlegen zu halten.

Analog können holographische Aufnahmen verschiedener Gegenstände bei jeweils verschiedenen Wellenlängen in einem einzigen, gegebenenfalls mehrschichtigen Hologramm festgehalten werden.

Die Tatsache, daß holographische Elemente im allgemeinen eine ausgeprägte Wellenlängenselektivität aufweisen kann für das erfindungsgemäße Informationsssystem auch nachteilig sein. Dies ist insbesondere dann der Fall, wenn ein polyspektrales Bild über ein holographisches Element erfaßt werden soll.

Bei einer von einem Menschen wahrzunehmenden Projektion findet die bekannte, auf die menschliche Wahrnehmung zurückzuführende Farbaddition von Licht unterschiedlicher Wellenlängen statt, die beispielsweise die Verwirklichung eines Vollfarbbildes aus den diskreten Grundfarben Rot, Grün und Blau ermöglicht. Der Umkehrschluß, daß ein Vollfarbbild sind in entsprechend diskreten Farbkomponenten "reduzieren" läßt, trifft im allgemeinen nicht zu. Die üblichen alltäglichen Geräte, die nach diesem Prinzip zu operieren scheinen, erfassen nicht nur Licht einer diskreten Wellenlängen sondern Licht eines ganzen Spektralbereichs.

Das hier zugrundeliegende Problem läßt sich in Analogie leichter vorstellen. Eine typische Szene strahlt polysektrales Licht ab. Dabei werden einzelne Photonen einer jeweiligen diskreten Wellenlänge, die einer jeweiligen diskreten Frequenz entspricht, abgestrahlt. Dies ist dem polyspektralen Klang eines Symphonieorchesters analog. Stellen Sie sich vor, wie ungenießbar die Musik wäre, wenn Sie lediglich Töne der genauen Frequenzen 440 Hz (der Kammerton A), 550 Hz (eine große Terz darüber) und 660 Hz (eine Quinte über dem A) wahrnehmen könnten. Der Effekt wäre umso drastischer, wenn die erste Geige gegen die internationale Norm ihre Geige mit einer Stimmgabel der Frequenz 441 Hz gestimmt hätte. Sie würde in dem Fall nur Stille erleben.

Glücklicherweise ist die Natur nicht so wählerisch bezüglich der benutzten Frequenzen wie ein Symphonieorchester. Eine typische Szene strahlt Photonen unzähliger Frequenzen ab. Zudem kann ein holographisches Element auch seine lichtbrechende Eigenschaft auf Lichtstrahlen auswirken, die in einem gewissen Bereich um die Wellenlänge des zur Herstellung benutzten Lichts liegt. Da dieser Bereich jedoch typischerweise sehr schmal ist, wird Lichtbrechung in diesem Bereich in der Anmeldung als Lichtbrechung "bei einer diskreten Wellenlänge" bezeichnet.

Die Anzahl der für eine ausreichende polyspektrale Lichterfassung notwendigen holographischen Element hängt somit vom Ziel der Lichterfassung ab. Erfindungsgemäß wird deshalb ein holographisches Element vorgesehen, das Licht bei einem oder einigen diskreten Wellenlängen bricht, das heißt bei weniger als 20, weniger als 10 oder weniger als 5 diskreten Wellenlängen. Werden zu viele holographische Aufnahmen in einem einzigen holographischen Element festgehalten, so führt dies zu einer gegenseitigen Beeinträchtigung deren Wirkung.

### 2.2 Markierungen

Vorzugsweise weist ein erfindungsgemäße holographische Element Markierungen auf. Dabei sind Markierungen als mindestens zwei Bereiche unterschiedlicher optischer Eigenschaften zu verstehen. Wird Licht sowohl über einen solchen Bereich als auch über mindestens einen solchen optisch unterschiedlichen Bereich erfaßt, so können die jeweilig erfaßten Lichtkenngrößen Unterschiede aufweisen, die auf die Markierung zurückzuführen sind. Da die jeweiligen Bereiche der Markierung vorzugsweise vorbestimmte optische Eigenschaften aufweisen, die das darauffallende Licht in einer entsprechend vorbestimmten Weise beeinflussen, kann das Vorhandensein einer Markierung anhand des erfaßten Lichts eindeutig festgestellt werden. Ebenfalls kann eine Kenngröße des erfaßten Licht in Relation zu einer vorbestimmten Beeinflussung dieser Kenngröße aufgrund der Markierung gemessen werden.

Somit können Markierungen verschiedenen Zwecken dienen. Zum Beispiel können Markierungen zur Bestimmung und/oder zur Eichung einer Kenngröße des projizierten bzw. erfaßten Lichts benutzt werden. Ebenfalls können Markierungen der Vermessung, Justage und/oder Eichung eines oder mehreren Komponenten, insbesondere einer Lichtleitvorrichtung, des erfindungsgemäßen Informationssystems dienen.

Eine Realisierung der Markierungen mittels des holographischen Elements bietet viele Vorteile. Vor allem lassen sich komplexe, optisch hochwertige Markierungen dadurch kostengünstig herstellen. Zudem kann es vorteilhaft sein, daß die Markierungen durch ihre Realisierung im holographischen Element im direkten Zusammenhang zu einer wesentlichen Lichtleitvorrichtung des Informationssystems stehen können. Des weiteren lassen sich komplexe Markierungen holographisch verwirklichen, die mit anderen Mitteln kaum oder gar nicht realisierbar wären. Nicht zuletzt sind holographische Markierungen von ihrer Natur aus wellenlängenselektiv, winkelselektiv und/oder phasenselektiv und somit zur Einsetzung als kennzeichnende bzw. selektive Elemente prädestiniert. Unter anderem können Markierung im holographischen Element realisiert werden, die beispielsweise lediglich unsichtbares Infrarotlicht beeinflussen.

Die Markierungen können eine beliebige Form aufweisen. Eine Ausführung in Form eines Fadenkreuzes wäre zum Beispiel bei einer Anwendung, der Markierungen als Bezugskoordinatensystem sinnvoll.

Die Markierungen können beliebige optischen Eigenschaften aufweisen. Eine zur Eichung einer Kombination einer Projektionsvorrichtung und einer Abtastvorrichtung geeignete Markierung könnte zum Beispiel spiegelnde Bereiche aufweisen, die von der Projektionsvorrichtung projizierte Strahlen vollständig auf die Abtastvorrichtung lenkt. Andere Bereiche könnten den Projektionsstrahl so lenken, daß er auf keinem Fall in die Abtastvorrichtung gelangt. Eine in diesem Zusammenhang vorteilhafte, mehrere Detektorbereiche aufweisende Abtastvorrichtung ist im Abschnitt 3.4 ( "besondere Abtastvorrichtungen") beschrieben.

### 2.3 ellipsoidartige Ausgestaltung

Vorzugsweise weist das holographische Element lichtbrechende Eigenschaften bei einer oder einigen diskreten Wellenlängen entlang einer Teilfläche eines virtuellen Ellipsoids auf, wobei das holographische Element und die Teilfläche vorzugsweise vor einem Auge gelagert ist und eines der Brennpunkte des virtuellen Ellipsoids mit den optischen Mittelpunkt des Auges übereinstimmt. Vorzugsweise stimmt der andere Brennpunkt des Ellipsoids mit einem optischen Ausgangspunkt einer Projektionsvorrichtung bzw. einem optischen Ausgangspunkt eines daran gekoppelten Lichtleitvorrichtung und/oder mit einem optischen Eingangspunkt einer Abtastvorrichtung bzw. einem optischen Eingangspunkt eines daran gekoppelten Lichtleitvorrichtung räumlich überein.

Bei räumlicher Übereinstimmung der virtuellen Brennpunkte des virtuellen Ellipsoids mit dem optischen Mittelpunkt des Auges und einem optischen Ausgangspunkt einer Projektionsvorrichtung bzw. einem optischen Ausgangspunkt eines daran gekoppelten Lichtleitvorrichtung, so ergibt sich bei entsprechend gewählten Brechungseigenschaften des virtuellen Ellipsoids einen vorteilhaften Strahlengang für die Projektion.

Stimmt der virtuellen Brennpunkte des virtuellen Ellipsoids mit dem optischen Mittelpunkt des Auges und einem optischen Eingangspunkt einer Abtastvorrichtung bzw. einem optischen Eingangspunkt eines daran gekoppelten Lichtleitvorrichtung räumlich überein, so ergibt sich bei entsprechend gewählten Brechungseigenschaften des virtuellen Ellipsoids einen vorteilhaften Strahlengang für die Abtastung. Dies gilt sowohl für die Abtastung aus dem Auge als auch für die Abtastung aus der Umgebung.

Die Ausführung einer lichtbrechenden Teilfläche eines virtuellen Ellipsoids durch ein holographisches Element weist insbesondere den Vorteil auf, daß das holographische Element selbst nicht ellipsoidförmig ausgestaltet sein muß. Dies ist insbesondere bei einer brillenartigen Ausgestaltung des erfindungsgemäßen Informationssystem von Vorteil. Zudem kann die durch das holographische Element hervorgerufene Lichtbrechung wellenlängenselektiv realisiert werden, so daß das holographische Element bei den restlichen Wellenlängen durchsichtig wirkt. Ähnlich könnte beispielsweise der Ellipsoid eines einer Abtastvorrichtung wellenlängenmäßig zuzuordnendes holographischen Elements andere Ausmaße und/oder eine andere Lage und/oder eine andere Ausrichtung als der Ellipsoid eines einer Projektionsvorrichtung wellenlängenmäßig zuzuordnendes holographischen Elements aufweisen. Dies wäre beispielsweise bei einer nicht konfokalen Anordnung einer Abtastvorrichtung und einer Projektionsvorrichtung sinnvoll.

### 2.3.linnenspiegelnd

Vorzugsweise weist das holographische Element lichtbrechende Eigenschaften bei einer oder einigen diskreten Wellenlängen auf, die einer Spiegelung an der Konkavseite einer gemäß der Krümmung eines rotationssymmetrischen Ellipsoids ausgebildeten Fläche entsprechen.

Gemäß der nachstehenden Erläuterung kann eine derartige Ausführung des holographischen Elements bei einer Projektion von Licht ins Auge bzw. einer Abtastung von Licht aus dem Auge vorteilhaft eingesetzt werden.

Wird das Auge als idealisiertes optisches System betrachtet, bei dem einfallendes Licht stets durch einen gemeinsamen optischen Mittelpunkt verläuft, und werden der optische Ausgang einer Projektionsvorrichtung bzw. einer daran gekoppelten Lichtleitvorrichtung und/oder der Eingang einer Abtastvorrichtung bzw. einer daran gekoppelten Lichtleitvorrichtung als Punktlichtquelle bzw. Punktdetektor betrachtet, so verlaufen von einem der Punkte ausgehende Strahlen stets zum anderen Punkt, wenn diese Punkte den jeweiligen Brennpunkten eines innenreflektierenden rotationssymmetrischen Ellipsoids entsprechen.

Mit einem holographischen Element lassen sich die Reflektionseigenschaften eines solchen Ellipsoids emulieren, ohne daß das holographische Element die äußerliche Form eines solchen Ellipsoids aufweisen muß. Des weiteren läßt sich mit Kenntnis der Form der Ellipsoid aus dem Einfallswinkel des Lichtes auf den Detektorpunkt den "Ausfallswinkel" des Lichtes aus dem Auge bzw. aus der Projektionsrichtung der Einfallswinkel auf das Auge ermitteln.

### 2.3.2senkrechtspiegelnd

Vorzugsweise weist das holographische Element lichtbrechende Eigenschaften bei einer oder einigen diskreten Wellenlängen auf, die einer Brechung an der Konkavseite einer gemäß der Krümmung eines rotationssymmetrischen Ellipsoids ausgebildeten Fläche entsprechen, welche Brechung einer Spiegelung an einer jeweiligen, um die Rotationsachse des Ellipsoids rotationssymmetrischen Kegelfläche entspricht, die am Ort der Brechung senkrecht zum Ellipsoid steht.

Gemäß der nachstehenden Erläuterungen kann eine derartige Ausführung des holographischen Elements bei einer Abtastung von Licht aus dem Gesichtsfeld eines Auges vorteilhaft eingesetzt werden.

Wird das Auge als idealisiertes optisches System betrachtet, bei dem einfallendes Licht stets durch einen gemeinsamen optischen Mittelpunkt verläuft, und wird der Eingang einer Abtastvorrichtung bzw. einer daran gekoppelten Lichtleitvorrichtung als Punktdetektor betrachtet, so verlaufen auf den einen Punkt gerichteten Strahlen stets durch den anderen Punkt, wenn diese Punkte den jeweiligen Brennpunkten eines wie oben beschriebenen rotationssymmetrischen Ellipsoids eines derart realisierten holographischen Elements entsprechen. Mit Kenntnis der Form der Ellipsoid läßt sich aus dem Einfallswinkel des Lichtes auf den Detektorpunkt den "Einfallswinkel" des Lichtes auf das Auge ermitteln.

Analog könnte ein solches holographisches Element in sekundären Sinne der Erfindung vorteilhaft dazu verwendet werden, Licht von einem Ausgangspunkt einer Projektionsvorrichtung bzw. einer daran gekoppelten Lichtleitvorrichtung in das Gesichtfeld des Auges zu projizieren.

Während ein solches holographisches Element durch die holographische Aufnahme eines reellen Gegenstands schwer zu verwirklichen wäre, läßt sich ein solches holographisches Element computergestützt realisieren. Das Ergebnis ist ein leicht reproduzierbares optisches Element, das beispielsweise in der Lage ist, auf ein Auge gerichtete Lichtstrahlen unter Beibehaltung einer eindeutigen Zuordnung zum Einfallswinkel auf einen gemeinsamen Punkt zu brechen. Zudem lassen sich mit einem holographischen Element die angegebenen Brechungseigenschaften ohne Auswirkungen auf die äußerliche Form des holographischen Elements emulieren.

Ein besonderer Vorteil einer wie hier beschriebenen Ausgestaltung des holographischen Elements (zwecks der hiesigen Erklärung: "HE1"-) liegt in ihrer möglichen Kombination mit einem holographischen Element ("HE2") gemäß dem vorhergehenden Abschnitt. Sind die jeweiligen Ellipsoide gleichförmig und virtuell ortsgleich, und wird HE1 zur Brechung von direkt aus der Umgebung fallenden Lichtstrahlen ("L1-Strahlen"), während HE2 zur Brechung von Lichtstrahlen ("L2-Strahlen") zwischen dem Auge und den projizierenden bzw. lichterfassenden Komponenten des Informationssystems, so sind diejenigen auf HE1 gerichtete L1-Strahlen und diejenigen auf/von HE2 gerichteten L2-Strahlen koaxial, die zwischen HE1 bzw. HE2 und der jeweiligen Projektions-, Abtast- bzw. Lichtleitvorrichtung den gleichen Strahlengang aufweisen. Dieser Vorteil wird unten unter Bezugnahme auf die Zeichnungen näher erläutert.

### 2.3.3Scanwinkelvergrößerung bzw. -verkleinerung

Vorzugsweise weist das holographische Element lichtbrechende Eigenschaften bei einer oder einigen diskreten Wellenlängen entlang einer Teilfläche eines virtuellen Ellipsoids auf, wobei das holographische Element dazu verwendet wird, den Scanwinkel einer Projektions- und/oder Abtastvorrichtung bzw. einer daran gekoppelten Lichtleitvorrichtung zu vergrößern oder zu verkleinern.

Wird die Projektions- bzw. Abtastrichtung einer Projektions- bzw. Abtastvorrichtung zeitlich verändert, ergibt sich ein Scanwinkel, das heißt ein zwischen jeweiligen Projektions- bzw. Abtastrichtungen gemessener Winkel. Eine solche Änderung der Projektions- bzw. Abtastrichtung erfolgt häufig durch eine steuerbare Lichtleitvorrichtung, die einen beschränkten Scanwinkelbereich oder eine beschränkte Scanwinkelauflösung aufweist.

Bei einer nicht kugelförmigen Ellipsoid ist der Winkel zwischen zwei von einem Brennpunkt des Ellipsoids ausgehenden Lichtstrahlen, die an der Innenfläche des Ellipsoids reflektiert werden, ungleich der Winkel zwischen diesen zwei Lichtstrahlen beim Eintreffen auf den anderen Brennpunkt des Ellipsoids.

Dementsprechend kann ein holographisches Element gemäß den vorhergehenden Abschnitten vorteilhaft dazu verwendet werden, den Scanwinkel einer Projektions- und/oder Abtastvorrichtung bzw. einer daran gekoppelten Lichtleitvorrichtung zu vergrößern oder zu verkleinern. Zudem vorteilhaft ist, daß die physikalische Form des holographischen Elements, keinesfalls mit der Form des aufgenommenen Gegenstands, das heißt der " virtuellen" Form des holographischen Elements, übereinstimmen muß. Dementsprechend können die Ausmaße des Ellipsoids gemäß den Systemerfordernissen frei gewählt werden.

### 2.4 Nachstellung

Vorzugsweise weist das erfindungsgemäße Informationssystem ein holographisches Element mit lichtbrechenden Eigenschaften bei einer oder einigen diskreten Wellenlängen, wobei die Lage des holographischen Elements und/oder des holographischen virtuellen Gegenstands bezüglich eines Teils des Informationssystems veränderbar ist. Dies gilt insbesondere im Fall eines holographischen Elements mit lichtbrechenden Eigenschaften bei einer oder einigen diskreten Wellenlängen entlang einer Teilfläche eines virtuellen Ellipsoids.

Aus der vorhergehenden Beschreibung wird ersichtlich, daß eine besondere Positionierung des holographischen Elements und/oder des holographischen virtuellen Gegenstands, insbesondere gegenüber dem Auge, einer Abtastvorrichtung, einer Projektionsvorrichtung und/oder einer daran gekoppelten Lichtleitvotrichtung erhebliche Vorteile erzielen kann.

Allerdings wird das Informationssystem üblicherweise nicht derart am Kopf fixiert, daß eine stets exakte Positionierung des holographischen Elements bzw. des holographischen virtuellen Gegenstands gegenüber dem Auge gewährleistet werden könnte. Zudem wird das Informationssystem üblicherweise Licht aus beiden Augen erfassen bzw. in beide Augen projizieren. Der Abstand zwischen beiden Augen kann jedoch bei verschiedenen Menschen sehr unterschiedlich sein.

Aus diesen und anderen Gründen ist es vorteilhaft, wenn die Lage des holographischen Elements und/oder des holographischen virtuellen Gegenstands bezüglich eines Teils des Informationssystems veränderbar ist. Die Lageveränderung kann ein-, zwei- oder dreidimensional erfolgt. Vorzugsweise ist jedoch die Lage des holographischen virtuellen Gegenstandes vorzugsweise bezüglich einer Abtastvorrichtung, einer Projektionsvorrichtung und/oder einer daran gekoppelten Lichtleitvorrichtung unveränderbar.

### 2.4.1 mechanische oder manuelle Nachstellung

Vorzugsweise ist die Lage des holographischen Elements und somit die Lage des holographischen virtuellen Gegenstands bezüglich eines Teils des Informationssystems mechanisch und/oder manuell veränderbar.

Eine mechanische Positionierung könnte vom Benutzer ausgelöst werden oder automatisch vom erfindungsgemäßen Informationssystem bei Bedarf und/oder beim Einschalten durchgeführt werden. Dabei könnte zum Beispiel verschiedene Positionen ausprobiert werden und die best funktionierende Position vom Benutzer identifiziert oder über die Abtastvorrichtung erkannt werden. Vorzugsweise umfaßt das Informationssystem Stellmotoren, Piezoelemente oder andere geeigneten mechanischen Vorrichtungen für die Positionierung.

Eine manuelle Positionierung könnte nach dem Zufallsprinzip oder beispielsweise anhand von sichtbaren Positionierhilfen durchgeführt werden. Solche Positionierhilfen könnten zum Beispiel die korrekte Lage des Informationssystems vor dem Auge andeuten.

Die Art und Weise der Veränderung hängt stark von der Gestaltung des Informationssystems ab und läßt sich deshalb hier im allgemeinen nicht weiter konkretisieren. Bei einem in der Form einer Brille ausgestalteten Informationssystem könnte die Veränderung beispielsweise darin liegen, daß eine linke und eine rechte Hälfte der Brille gegeneinander verschoben werden. Ebenfalls könnten als holographisches Element ausgeführte "Brillengläser" gegenüber dem Gestell verschoben werden.

### 2.4.2 optische oder elektronische Nachstellung

Vorzugsweise ist die Lage des virtuellen Gegenstands bezüglich eines Teils des Informationssystems optisch quasi veränderbar. Alternativ ist die Lage des holographischen virtuellen Gegenstands bezüglich eines Teils des Informationssystems vorzugsweise elektronisch veränderbar.

Eine quasi Veränderung der Lage des holographischen virtuellen Gegenstands ließe sich durch ein holographisches Element realisieren, das mehrere, jeweilig verschobene Aufnahmen des Gegenstands unter jeweilig unterschiedlichen holographischen Bedingungen aufweist.

Zum Beispiel könnte der Gegenstand mehrfach bei jeweilig unterschiedlichen Wellenlängen und bei jeweilig unterschiedlichen Positionen aufgenommen werden. Wird dann die Wellenlänge des über das holographische Element erfaßten bzw. projizierten Lichts entsprechend geändert, so wird das Licht an einem anders positionierten holographischen virtuellen Gegenstand virtuell gebrochen, was einer Lageveränderung des holographischen virtuellen Gegenstands entspricht.

Eine elektronische Veränderung der Lage des holographischen virtuellen Gegenstands setzt die Verwendung eines elektro-holographischen Elements als holographisches Element vorraus. Das elektro-holographische Elemente könnte beispielsweise mehrere, jeweilig verschobene Aufnahmen des Gegenstands aufweisen, welche Aufnahmen durch das Anlegen einer geeigneten Spannung jeweilig aufrufbar sind.

Eine wie oben beschriebene Positionierung des holographischen virtuellen Gegenstands könnte vom Benutzer ausgelöst werden oder automatisch vom erfindungsgemäßen Informationssystem bei Bedarf und/oder beim Einschalten durchgeführt werden. Dabei könnte zum Beispiel verschiedene Positionen ausprobiert werden und die best funktionierende Position vom Benutzer identifiziert oder über die Abtastvorrichtung erkannt werden.

Die oben genannten optischen und elektro-holographischen Verfahren zur Veränderung der Lage eines holographischen virtuellen Gegenstands könnte analog dazu verwendet werden, die Lichtbrechungseigenschaften des holographischen Element auf eine vorbestimmte Art und Weise zu ändern.

### 2.5 Strahlen senkrecht zum Auge

Vorzugsweise umfaßt das erfindungsgemäße Informationssystem ein holographisches Element, das in der Lage ist, Lichtstrahlen gemäß ihrer Ausfallswinkel aus einer Projektionsvorrichtung oder einer daran gekoppelten Lichtleitvorrichtung senkrecht auf ein jeweiliges Gebiete des Auges und/oder von einem jeweiligen Gebiete des Auges senkrecht austretende bzw. zurückreflektierte Lichtstrahlen mit einer entsprechenden Einfallswinkel in eine Abtastvorrichtung oder eine daran gekoppelte Lichtleitvorrichtung zu lenken.

Die Vorzüge und mögliche Gestaltungskriterien eines solchen holographischen Elements wurden in Abschnitt 1.6.2 ausführlich behandelt. Als solches holographische Element kann sich insbesondere ein holographisches Element gemäß Abschnitt 2.3 bei entsprechender Positionierung und Krümmung des virtuellen Ellipsoids eignen.

### 3 Projektion und Abtastung

Nachstehend werden einige bevorzugte Merkmale beschrieben, die der Projektion, der Abtastung sowie dazugehörigen Vorrichtungen betreffen.

### 3.1 Strahldurchmesser

Die Optik der Projektions- bzw. Abtastvorrichtung ist vorzugsweise derart ausgelegt, daß ein Lichtstrahl mit vorbestimmtem oder bestimmbarem Strahldurchmesser projiziert bzw. erfaßt wird. Diese (Vor-)Bestimmung des Strahldurchmessers kann über eine geeignete Lichtgestaltungsvorrichtung, beispielsweise über eine Fokussier- oder Blendvorrichtung verwirklicht werden.

Die Bestimmung des Strahldurchmessers beispielsweise in Verbindung mit einer Bestimmung der Projektions- bzw. Abtastrichtung erlaubt eine räumliche gezielte Projektion bzw. Abtastung.

Unter dem Begriff "schmaler Lichtstrahl" ist sowohl hier als auch an anderen Stellen der Beschreibung vorzugsweise ein Lichtstrahl gemäß DE 101 27 826 zu verstehen, der so mit geringer Divergenz, geringer Konvergenz oder kohärent in das Auge projiziert wird, daß der Lichtstrahl an dem Luft-Augapfel-Übergang, insbesondere an dem Luft-Kornea-Übergang einen im Vergleich zum Pupillendurchmesser unwesentlichen Durchmesser, beispielsweise unter 100 µm, unter 50 µm, unter 10 µm oder gar unter 5 µm, hat.

Die Verwendung eines schmalen Lichtstrahls bringt systemtechnisch den Vorteil, daß der Strahlgang des gesamten Lichtstrahls näherungsweise den gleichen Strahlengang wie der dem Lichtstrahl zugeordneten Hauptstrahl hat. Das heißt, die Brechung des Lichtstrahls an einer unebenen Fläche führt zu keiner wesentlichen makroskopischen Änderung der Divergenz, der Konvergenz oder der Kohärenz des Lichtstrahls. Diese gilt sowohl für die Projektion als auch für die Abtastung.

Durch die Verwendung einer mit entsprechend hoher Lichtstärke strahlenden Projektionsvorrichtung, beispielsweise einer LED oder Laserdiode, läßt sich bei einer Projektion ohne weiteres eine ausreichende Menge Licht trotz des schmalen Lichtstrahls auf den zu beleuchtenden Teil des Auges bringen.

### 3.2 Fokussierung

Vorzugsweise verfügt die erfindungsgemäße Projektions-und/oder Abtastvorrichtung über eine Fokussiervorrichtung, über die der projizierte bzw. erfaßte Lichtstrahl fokussierbar ist.

Eine Fokussiervorrichtung kann, wie oben beschrieben, einer räumlichen gezielten Projektion bzw. Abtastung dienen. Insbesondere kann eine Fokussiervorrichtung dazu eingesetzt werden, Gegenstände, die sich in einem über die Fokussiervorrichtung bestimmbaren Entfernung entlang dem Abtaststrahlengang von der Abtastvorrichtung befinden, scharf auf die detektierenden Elemente der Abtastvorrichtung abzubilden. Analog kann eine Fokussiervorrichtung . projizierte Lichtstrahlen auf Gegenstände fokussieren, d.h. zur Konvergenz bringen, die sich in einem über die Fokussiervorrichtung bestimmbaren Entfernung entlang dem Projektionsstrahlengang von der Projektionsvorrichtung befinden.

Erfindungsgemäß kann eine Fokussiervorrichtung somit zum Beispiel dazu angewandt werden, Licht auf ein bestimmtes Teil des Auge zu projizieren bzw. von einem bestimmten Teil des Auges zu erfassen. Dies kann insbesondere zur gezielten Abtastung einer von mehreren "hintereinander liegenden" okularen Strukturen, wie dies beispielsweise bei der vorderen Korneafläche, der hinteren Korneafläche und der Netzhaut der Fall ist. Dies gilt analog für die Projektion.

Bei sehr schmalen, quasi konvergenten Lichtstrahlen, deren Strahlendurchmesser sich im Laufe des Strahlengangs kaum ändert, ist eine Fokussierung begrenzt zweckmäßig.

### 3.3 gemeinsamer Strahlengang

Vorzugsweise umfaßt das erfindungsgemäße Informationssystem eine Projektionsvorrichtung und eine Abtastvorrichtung, die einen gemeinsamen Strahlengang aufweisen. Die Projektionsvorrichtung und die Abtastvorrichtung sind vorzugsweise konfokal, beispielsweise auf jeweiliger Seiten eines Teilerspiegels angeordnet.

Haben eine Projektionsvorrichtung und eine Abtastvorrichtung einen gemeinsamen Strahlengang, so können sie aufgrund der Reversibilität eines Lichtstrahls eine eventuell vorhandene Lichtleitvorrichtung gemeinsam verwenden. Dies stellt eine Vereinfachung des Informationssystems dar.

Gleichwohl gewährleistet ein gemeinsamer Strahlenweg, daß projizierte Lichtstrahlen, die entgegen die Projektionsrichtung beispielsweise vom Auge zurückgestrahlt werden, sozusagen "automatisch" der Abtastvorrichtung zugeleitet werden. Somit kann eine aufwändige "Synchronisierung" einer der Abtastvorrichtung zugeordneten Lichtleitvorrichtung und einer der Projektionsvorrichtung zugeordneten Lichtleitvorrichtung entfallen.

Wird eine konfokale Anordnung der Projektions- und der Abtastvorrichtung über einen Teilerspiegel oder ein ähnliches strahlteilendes Element realisiert, so ist es vorteilhaft, dem Abtaststrahl dem Vorrang zu geben. Dies ist insbesondere bei der Abtastung schwach abstrahlender Gegenstände, beispielsweise der Netzhaut, von Vorteil. Vorzugsweise leitet der Teilerspiegel mehr als 95% des Abtaststrahls in Richtung Abtastvorrichtung. Anstatt 95% kann zu Günsten des Projektionsstrahls lediglich mehr als 90%, 85% oder gar 80% des Abtaststrahls in Richtung Abtastvorrichtung vom Teilerspiegel weitergeleitet werden. Die dadurch verursachte Abschwächung des Projektionsstrahls ist üblicherweise unbedeutend, da dies durch eine entsprechende Erhöhung der Intensität des von der Projektionsvorrichtung projizierten Lichtstrahls ausgeglichen werden kann. Die maximale Intensität des Projektionsstrahls wird typischerweise durch die photonische Belastbarkeit des Augengewebes bestimmt. Ist eine Erhöhung der Intensität des Projektionstrahls bei einer gewünschten Ausführung des Informationssystem nicht zweckmäßig, so kann ein beliebiges anderes Lichtteilungsverhältnis des Teilerspiegels gewählt werden.

### 3.4 besondere Abtastvorrichtungen

Vorzugsweise weist das erfindungsgemäße Informationssystem eine Abtastvorrichtung auf, die Licht in mindestens zwei benachbarten, vorzugsweise konzentrisch umeinander liegenden, Bereichen erfaßt.

Durch eine Abtastvorrichtung, die Licht in mindestens zwei benachbarten, nicht umeinander liegenden Bereichen erfaßt, läßt sich feststellen, in welche Richtung ein darauffallender Lichtstrahl sich bewegt und/oder wann der Lichtstrahl einen bestimmten Bereich verläßt. Dies wird beispielsweise durch einen Vergleich der Intensität des auf das jeweilige Gebiete fallenden Lichtstrahls.

Durch eine Abtastvorrichtung, die Licht in mindestens zwei konzentrisch umeinander liegenden Bereichen erfaßt, läßt sich, wie oben beschrieben, feststellen, ob ein darauffallender Lichtstrahl sich in Richtung Detektormitte oder von der Detektormitte hinweg bewegt und/oder wann der Lichtstrahl einen bestimmten Bereich verläßt..

Da die obengenannte Erkenntnisse ein Lokalisierung des Strahlendpunkts erlauben, können sie beispielsweise bei Kenntnis der geometrischen Anordnung der Abtastvorrichtung, der Lichtquelle und der am Strahlengang beteiligten Lichtleitvorrichtungen für die Eichung und/oder Steuerung der Lichtleitvorrichtungen des Informationssystems vorteilhaft eingesetzt werden.

Insbesondere kann eine Abtastvorrichtung, die Licht in mindestens zwei konzentrisch umeinander liegenden Bereichen erfaßt, vorteilhafterweise dazu angewandt werden, ein Strahlende bezüglich zweier Koordinaten zu orten bzw. positionieren.

### 3.5 Nachstellung

Analog den im Abschnitt 2.4 beschriebenen Maßnahmen, kann die Lage, Orientierung und/oder die optischen Eigenschaften anderer vom erfindungsgemäßen Informationssystem umfaßten optischen Vorrichtung, insbesondere eventueller Lichtleitvorrichtungen, verändert werden.

### 3.6 Scannervorrichtung

Vorzugsweise umfaßt die Projektions- bzw. Abtastvorrichtung eine steuerbare Lichtleitvorrichtung, die die Richtung ändert, in die ein Lichtstrahl projiziert, bzw. aus welcher Richtung ein Lichtstrahl erfaßt wird. Solche Lichtleitvorrichtungen, teilweise unter dem Begriff "Scanner" bekannt, sind dem Fachmann bekannt und umfassen verschiedenstartige Vorrichtungen. Hierzu gehören beispielsweise elektromechanische, akustomechanische und ähnliche bewegliche Spiegelvorrichtungen, elektro- und akustooptische Modulatoren, elektroholographische Elemente, bewegliche Lichtleiteranordnungen u.s.w.

### 3.7 besondere Lichtquellen

Vorzugsweise umfaßt die Projektionsvorrichtung einen Laser, der Licht bei einem oder mehreren von mehreren regelmäßig verteilten Wellenlängen projizieren kann.

In letzter Zeit sind Laser, die regelmäßig verteilgte Moden aufweisen, entwickelt worden. Ein derartiger Laser eignet sich hervorragend zur Verwendung in Kombination mit einem holographischen Element, daß Licht bei mehreren regelmäßig verteilten diskreten Wellenlänge bricht.

Die vorliegende Erfindung wird nachstehend anhand der Beschreibung von Ausführungsbeispielen unter Bezugnahme auf die Zeichnungen näher beschrieben. Es zeigen:
Fig. 1 eine schematische Darstellung eines abtastenden Informationssystems gemäß einem ersten Ausführungsbeispiel;
Fig. 2 eine schematische Darstellung eines projizierenden Informationssystems gemäß einem zweiten Ausführungsbeispiel;
Fig. 3 eine schematische Darstellung eines Informationssystems gemäß einem dritten Ausführungsbeispiel;
Fig. 4 eine schematische Darstellung eines Informationssystems gemäß einem vierten Ausführungsbeispiel;
Fig. 5 eine schematische Darstellung eines Informationssystems gemäß einem fünften Ausführungsbeispiel;
Fig. 6 eine schematische Detailansicht eines Informationssystems gemäß einem sechsten Ausführungsbeispiel;
Fig. 7A eine schematische Draufsicht auf ein Informationssystem gemäß einem siebten Ausführungsbeispiel;
Fig. 7B eine schematische Seitenansicht auf ein Informationssystem gemäß einem siebten Ausführungsbeispiel;
Fig. 8A eine schematische Vorderansicht auf ein Informationssystem gemäß einem achten Ausführungsbeispiel;
Fig. 8B eine schematische Draufsicht auf ein Informationssystem gemäß einem achten Ausführungsbeispiel entlang Schnitt B in Figur 8A;
Fig. 8C eine schematische Draufsicht auf ein Informationssystem gemäß einem achten Ausführungsbeispiel entlang Schnitt C in Figur 8A;
Fig. 9A-9E eine schematische Darstellung der Funktionsweise einer Abtastvorrichtung gemäß einem neunten Ausführungsbeispiel; und
Fig. 10A-10D eine schematische Darstellung der Funktionsweise einer Abtastvorrichtung gemäß einem zehnten Ausführungsbeispiel.

In der Beschreibung der Figuren werden ähnliche oder identische Gegenstände mit ähnlich oder gleich endenden Bezugsziffern bezeichnet. Viele der abgebildeten Gegenstände weisen symmetrische oder komplementäre Komponenten auf, die durch einen Zusatzbuchstaben, beispielsweise "L" für links und "R" für rechts, nach dem Bezugsziffer unterschieden werden. Betrifft die Aussage jede einzelne Komponente einer solchen symmetrischen oder komplementären Gruppierung, wird auf den Zusatzbuchstaben in manchen Fällen der Übersichtlichkeit halber verzichtet.

### Fig. 1

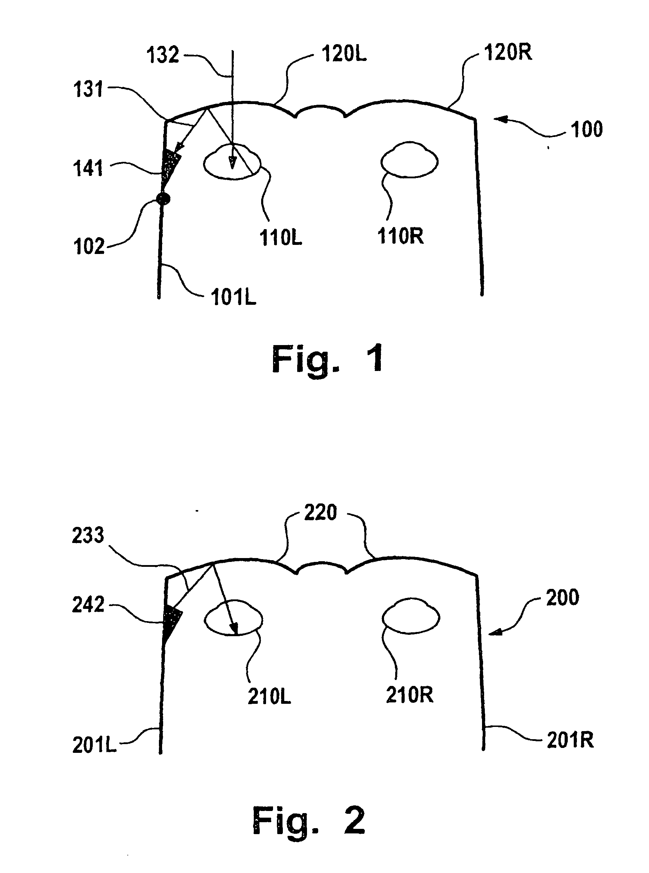

Figur 1 zeigt eine schematische Darstellung eines abtastenden Informationssystems 100 gemäß einem ersten Ausführungsbeispiel. Dabei ist das abtastende Informationssystem 100 in der Form einer Brille 100 gestaltet. Die Brille 100 weist zwei als Brillengläser 120R, 120L ausgeführte holographische Elemente 120R, 120L auf, die jeweils vor einem Auge 110R, 110L angeordnet sind. Am linken Bügel 101L der Brille 100 ist eine Abtastvorrichtung 141 befestigt, die in der Lage ist, vom Auge 110 kommende Lichtstrahlen 131 zu erfassen. Vorzugsweise umfaßt die Abtastvorrichtung 141 eine nicht dargestellte Lichtleitvorrichtung, beispielsweise eine Scannervorrichtung, die momentane Erfassungsrichtung der Abtastvorrichtung 141 bestimmt und zeitlich gemäß einem vorbestimmten Scanmuster verändert. Selbstverständlich könnte eine weitere, nicht dargestellte Abtastvorrichtung ebenfalls am rechten Bügel 101R befestigt sein. Der Lichtstrahl 132 deutet an, daß aus der Umgebung stammende Lichtstrahlen 132 vom holographischen Element 120 ungehindert durchgelassen werden.

In diesem Ausführungsbeispiel dient das holographische Element 120 lediglich dazu, vom Auge 110 kommende Lichtstrahlen 131 in Richtung Abtastvorrichtung 141 zu brechen. Die zu brechenden Wellenlängen hängt von der gewünschten Erfassung ab. Sollte zum Beispiel ein Bild der Netzhautstruktur erfaßt werden, wäre eine Lichtbrechung des holographischen Elements bei einer diskreten Wellenlänge im Infrarotbereich angebracht. Sollte ein Bild der Irisstruktur erfaßt werden, wäre eine Lichtbrechung des holographischen Elements bei einigen diskreten Wellenlänge im sichtbaren Bereich sinnvoll.

Um eine vorbestimme Relativanordnung der Abtastvorrichung 141 zum holographischen Element 120 zu gewährleisten, kann beispielsweise das Scharnier 102 zum Zusammenklappen des Brillengestells hinter der Abtastvorrichtung 141 angeordnet sein.

### Fig. 2

Figur 2 zeigt eine schematische Darstellung eines projizierenden Informationssystems 200 gemäß einem zweiten Ausführungsbeispiel. Dabei ist das projizierende Informationssystem 200 in der Form einer Brille 200 gestaltet. Die Brille 200 weist zwei als Brillengläser 220 ausgeführte holographische Elemente 220 auf, die jeweils vor einem Auge 210 angeordnet sind. Am linken Bügel 201L der Brille 200 ist eine Projektionsvorrichtung 242 befestigt, die in der Lage ist, Lichtstrahlen 233 auf bzw. in das Auge 210 zu projizieren. Vorzugsweise umfaßt die Projektionsvorrichtung 242 eine nicht dargestellte Lichtleitvorrichtung, beispielsweise eine Scannervorrichtung, die momentane Projektionsrichtung der Projektionsvorrichtung 242 bestimmt und zeitlich gemäß einem vorbestimmten Projektionsmuster verändert. Selbstverständlich könnte eine weitere, nicht dargestellte Projektionsvorrichtung ebenfalls am rechten Bügel 201R befestigt sein.

In diesem Ausführungsbeispiel dient das holographische Element 220 lediglich dazu, von der Projektionsvorrichtung 242 projizierte Lichtstrahlen 233 in Richtung Auge 210 zu brechen. Die vom holographischen Element 220 zu brechenden Wellenlängen werden deshalb bevorzugt an die Wellenlänge der Projektionsstrahlen 233 angepaßt sein.

### Fig. 3

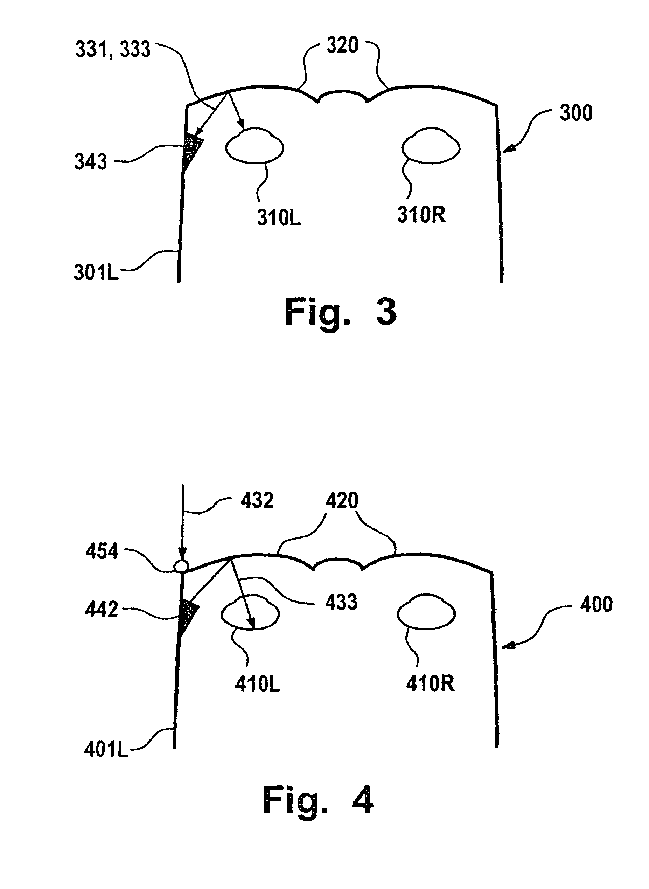

Figur 3 zeigt eine schematische Darstellung eines Informationssystems 300 gemäß einem dritten Ausführungsbeispiel. Dabei ist das Informationssystem 300 in der Form einer Brille 300 gestaltet. Die Brille 300 weist zwei als Brillengläser 320 ausgeführte holographische Elemente 320 auf, die jeweils vor einem Auge 310 angeordnet sind. Am linken Bügel 201L der Brille 200 ist eine kombinierte Projektions- und Abtastvorrichtung 343 befestigt, die in der Lage ist, sowohl Lichtstrahlen 333 auf bzw. in das Auge 310 zu projizieren als auch vom Auge 310 kommende Lichtstrahlen 331 zu erfassen. Vorzugsweise umfaßt die Projektionsvorrichtung 343 eine nicht dargestellte Lichtleitvorrichtung, beispielsweise eine Scannervorrichtung, die momentane Projektionsrichtung der Projektionsvorrichtung 343 bestimmt und zeitlich gemäß einem vorbestimmten Projektionsmuster verändert.

Ein solches Informationssystem 300 könnte zum Beispiel dazu dienen, die Netzhaut des Auges 310 aktiv punkuell zu beleuchten, und das von der Netzhaut zureflektierte Licht 331 zwecks einer Erkennung von Netzhautstrukturen erfassen.

In diesem Ausführungsbeispiel bricht das holographische Element 320 sowohl das von der Projektionsvorrichtung 343 projizierte Licht 333 als auch das von der Abtastvorrichtung 343 erfaßte Licht 331.

### Fig. 4

Figur 4 zeigt eine schematische Darstellung eines Informationssystems 400 gemäß einem vierten Ausführungsbeispiel. Dabei ist das Informationssystem 400 in der Form einer Brille 400 gestaltet. Die Brille 400 weist zwei als Brillengläser 420 ausgeführte holographische Elemente 420 auf, die jeweils vor einem Auge 410 angeordnet sind. Am linken Bügel 401L der Brille 400 ist eine Projektionsvorrichtung 442 befestigt, die in der Lage ist, Lichtstrahlen 433 auf bzw. in das Auge 410 zu projizieren. Dabei bricht das holographische Element 420 das von der Projektionsvorrichtung 442 projizierte Licht 433 in Richtung Auge 410. Des weiteren ist am vorderen Teil des linken Bügels 401L eine Abtastvorrichtung 454, beispielsweise eine Kamera 454, angeordnet, die Lichtstrahlen 432 aus der Umgebung erfaßt.

Ein solches Informationssystem 400 könnte zum Beispiel dazu dienen, Informationen in Korrelation mit dem wahrgenommenen Gesichtsfeld des Auges 410 in das Auge 410 zu projizieren.

### Fig. 5

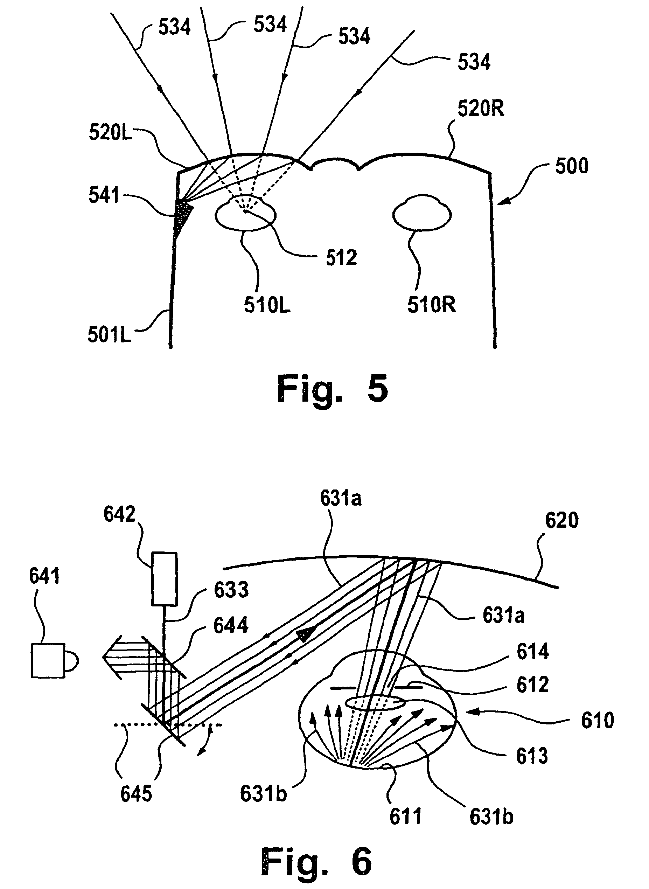

Figur 5 zeigt eine schematische Darstellung eines Informationssystems 500 gemäß einem fünften Ausführungsbeispiel. Dabei ist das Informationssystem 500 in der Form einer Brille 500 gestaltet. Die Brille 500 weist zwei als Brillengläser 520 ausgeführte holographische Elemente 520 auf, die jeweils vor einem Auge 510 angeordnet sind. Am linken Bügel 501L der Brille 500 ist eine Abtastvorrichtung 541 befestigt, die in der Lage ist, vom holographischen Element auf sie gebrochene Lichtstrahlen 532 aus der Umgebung zu erfassen.

Das holographische Element 520 ist derart ausgeführt, daß auf den optischen Mittelpunkt 512 des Auges 510 gerichtete Lichtstrahlen 534 aus der Umgebung auf einen gemeinsamen Punkt gebrochen werden. An diesem Punkt ist der optische Eingang der Abtastvorrichtung 541 angeordnet. Vorzugsweise werden die Lichtstrahlen 534 vom holographischen Element 520 derart gebrochen, daß deren jeweiliger Einfallswinkel auf die Abtastvorrichtung 541 eindeutige Auskunft über den Einfallswinkel des jeweiligen Lichtstrahls auf das holographische Element 520 liefert. Eine mögliche Ausführungsform eines solchen holographischen Elements 520 ist im Abschnitt 2.3.2 beschrieben.

Ein solches Informationssystem 500 könnte zum Beispiel dazu dienen, Informationen bezüglichen des vom Auge 510 wahrgenommenen Gesichtsfelds zu gewinnen.

### Fig. 6

Figur 6 zeigt eine schematische Detailansicht eines Informationssystems 600 gemäß einem sechsten Ausführungsbeispiel. Das Informationssystem 600 weist eine Projektionsvorrichtung 642, eine Abtastungvorrichtung 641, eine Lichtleitvorrichtung 645 sowie ein vor einem Auge 610 angeordnetes holographisches Element 620. Die Projektionsvorrichtung projiziert ein Lichtstrahl 633, der ein Teilerspiegel 644 teilweise passiert und von der Lichtleitvorrichtung 645 über das holographische Element 620 auf die Netzhaut 611 des Auges 610 gelenkt wird. Dort wird der projizierte Lichtstrahl 633 als Reflexlichtstrahlen 631 in verschiedene Richtungen zurückgestreut. Manche 631a der Reflexlichtstrahlen 631 werden von der Linse 613 des Auges 610 derart gebündelt, daß sie 631a nahezu parallel jedoch entgegengesetzt zum Projektionsstrahl 633 durch die Pupille 614 hindurchstrahlen. Danach werden diese Reflexlichtstrahlen 631a vom holographischen Element 620 gebrochen, von der Lichtleitvorrichtung 645 gelenkt, am Teilerspiegel 644 teilweise reflektiert und von der Abtastvorrichtung 641 erfaßt. Andere 631b der Reflexlichtstrahlen 631 werden beispielsweise von der Iris 612 des Auges 610 an einem Austreten aus dem Auge 610 gehindert.

In diesem Ausführungsbeispiel teilen sich der Projektionsstrahl 633 und der Abtaststrahl 631a einen gemeinsamen Strahlengang. Somit sind keine zwei Lichtleitvorrichtungen 645 notwendig. Da das Netzhaut lediglich ca. 4% bis 10% des darauffallenden Lichtes reflektiert, wird vorzugsweise ein Teilerspiegel 644 gewählt, der den Abtaststrahl 631a nahezu vollständig in Richtung Abtastvorrichtung 641 reflektiert und den Projektionsstrahl 633 dementsprechend schwächt. Um diese Abschwächung des Projektionsstrahls 633 zu kompensieren, wird die Intensität des Projektionsstrahls 633 entsprechend erhöht. Die maximale Intensität des Projektionsstrahls 633 wird typischerweise durch die maximale Belastungsgrenze der Netzhaut 611 bestimmt.

In einem solchen Ausführungsbeispiel kann die Einschränkung des abgetasteten Gebiets über eine entsprechende räumliche Einschränkung des vom Projektionsstrahl 633 beleuchteten Gebiets erfolgen. Eine Einschränkung der Strahlengänge, entlang welchen ein Lichtstrahl 631a von der Netzhaut 611 zur Abtastvorrichtung 641 zurückgestrahlt werden kann, erfolgt automatisch durch die Pupille 614.

### Fig. 7

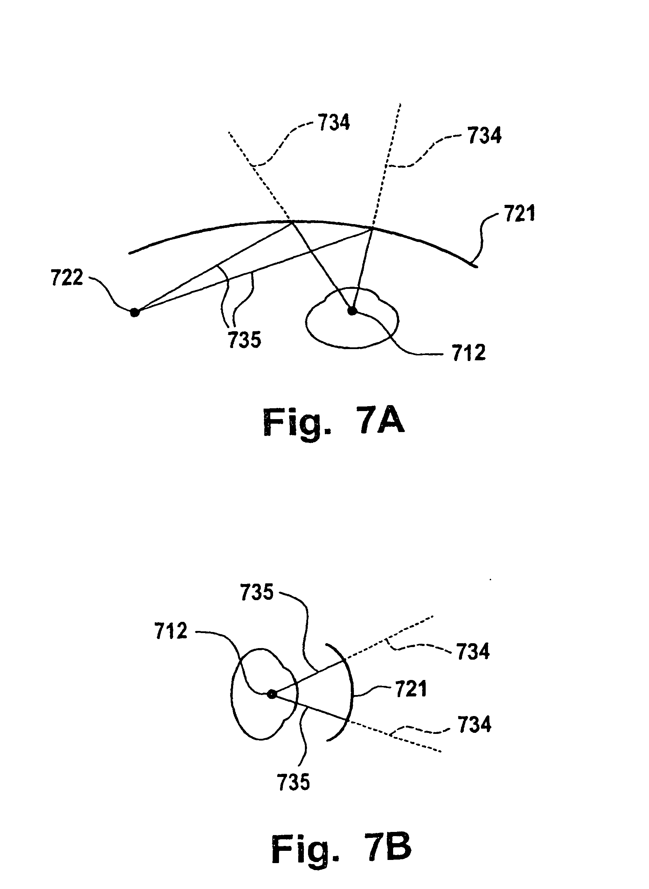

Fig. 7A und 7B zeigen eine schematische Draufsicht bzw. Seitenansicht auf ein Informationssystem gemäß einem siebten Ausführungsbeispiel, bei dem das holographische Element Licht entlang einer virtuellen Ellipsoidfläche 721 bricht.

In den Figuren 7A und 7B ist schematisch dargestellt, wie auf das optische Augenmittelpunkt 712 gerichtete Lichtstrahlen 734, die an der vituellen Ellipsoidfläche gemäß einer an der Reflektionsstelle senkrecht zur Ellipsoidfläche 721 stehenden spiegelnden Fläche reflektiert werden, durch einen gemeinsamen Punkt 722 verlaufen, der auch von denjenigen Strahlen 735 gemeinsam durchlaufen wird, die vom optischen Augenmittelpunkt 712 ausgehend an der Innenseite der Ellipsoidfläche 721 spiegelnd reflektiert werden.

### Fig. 8

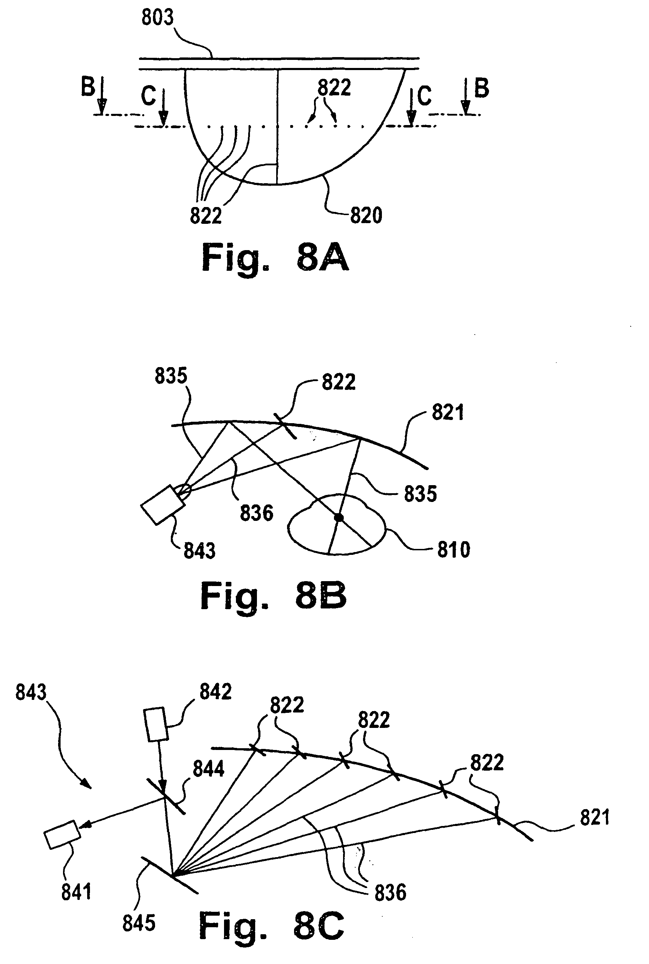

Figur 8A zeigt eine schematische Vorderansicht auf ein Informationssystem gemäß einem achten Ausführungsbeispiel. Figur 8B zeigt eine schematische Draufsicht entlang Schnitt B in Figur 8A. Figur 8C zeigt eine schematische Draufsicht entlang Schnitt C in Figur 8A.

Figur 8A zeigt ein in der Form eines Brillenglases 820 ausgeführtes holographisches Element 820, das an einem Brillengestell 803 befestigt ist. Das holographische Element 820 weist indirekt Markierungen 822 auf.

Vorzugsweise sind die Markierungen 822 nicht im allgemeinen sichtbar, sondern sind holographisch realisiert, so daß sie lediglich Licht einer bestimmten Wellenlänge, Phase und Einfallswinkel beeinflussen.

Figur 8B zeigt eine kombinierte Projektions- und Abtastvorrichtung 843, die Lichtstrahlen 835, 836 auf einen Markierungen 822 aufweisenden virtuellen holographischen Gegenstand 821 projiziert. Projizierte Lichtstrahlen 835, die die Markierungen nicht treffen, werden vom virtuellen holographischen Gegenstand 821 in Richtung Auge 810 gebrochen. Projizierte Lichtstrahlen 836, die die Markierungen treffen, werden vom virtuellen holographischen Gegenstand 821 entlang dem Projektionsstrahlengang zurückreflektiert und können somit von der kombinierte Projektions- und Abtastvorrichtung 843 erfaßt werden. So kann sowohl die Projektionsrichtung als auch der aufgrund einer Projektion und anschließender Erfassung des projizierten Lichtstrahls zu erwartende Signalpegel überprüft werden. Zudem können die erkannten Markierungen 822 als Bezugskoordinaten bei einer Informationsgewinnung oder einem Zurverfügungstellen von Informationen Verwendung finden.

Figur 8C zeigt den möglichen Aufbau einer kombinierten Projektions- und Abtastvorrichtung 843, die ähnlich der Figur 6 eine Projektionsvorrichtung 842, eine Abtastvorrichtung 841, einen Teilerspiegel 844 und eine Lichtleitvorrichtung 845 umfaßt. Über die analog der Figur 8B projizierten und von den Markierungen 822 des virtuellen holographischen Gegenstands 821 zurückreflektierten Lichtstrahlen 836 läßt sich beispielsweise der Stellwinkel der Lichtleitvorrichtung 845 überprüfen. Zudem können die erkannten Markierungen 822 als Bezugskoordinaten bei einer Informationsgewinnung oder einem Zurverfügungstellen von Informationen Verwendung finden.

### Fig. 9

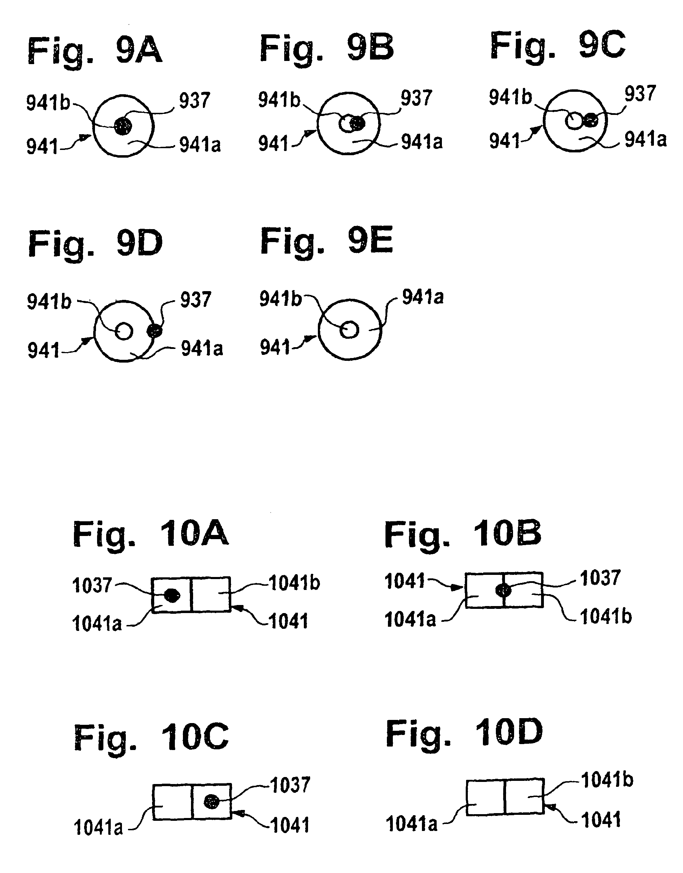

Die Figuren 9A-9E zeigen eine schematische Darstellung der Funktionsweise einer Abtastvorrichtung 941 gemäß einem neunten Ausführungsbeispiel, wobei die Abtastvorrichtung 941 zwei konzentrisch umeinander liegende detektierende Bereiche 941a, 941b umfaßt.

In Figur 9A detektiert lediglich Bereich 941b Licht. Somit muß das Strahlende gänzlich innerhalb des Bereichs 941b liegen. Anschließend detektieren beide Bereiche 941a und 941b Licht gemäß Figur 9B. Das Strahlende bewegt sich eindeutig vom Bereich 941b hinweg. Die Bewegungsrichtung ist unbekannt.

Anschließend detektiert lediglich Bereich 941a Licht gemäß Figur 9C. Somit muß das Strahlende gänzlich innerhalb des Bereichs 941a liegen. Anschließend gemäß Figur 9D detektiert Bereich 941b kein Licht, während Bereich 941a weniger Licht als zuvor detektiert. Das Strahlende bewegt sich eindeutig der Abtastvorrichtung 941 hinweg. Die Bewegungsrichtung ist unbekannt. In Figur 9E detektiert weder Bereich 941a noch Bereich 941b Licht. Das Strahlende liegt somit eindeutig außerhalb der Bereiche 941a und 941b.

Eine derartige Abtastvorrichtung 941 kann vorteilhafterweise dazu angewandt werden, ein Strahlende bezüglich zweier Koordinaten zu orten bzw. positionieren.

### Fig. 10

Die Figuren 10A-10D zeigen eine schematische Darstellung der Funktionsweise einer Abtastvorr.ichtung 1041 gemäß einem zehnten Ausführungsbeispiel, wobei die Abtastvorrichtung 1041 Licht in zwei benachbarten Bereichen 1041a, 1041b detektiert.

In Figur 10A detektiert lediglich Bereich 1041a Licht. Somit muß das Strahlende zumindest teilweise innerhalb des Bereichs 1041a liegen. Anschließend detektieren beide Bereiche 1041a und 1041b Licht gemäß Figur 10B. Das Strahlende bewegt sich eindeutig vom Bereich 1041a hinweg in Richtung des Bereichs 1041b.

Anschließend detektiert lediglich Bereich 1041b Licht gemäß Figur 10C. Somit muß das Strahlende zumindest teilweise innerhalb des Bereichs 1041b liegen und sich weiter in die bisher bestimmte Richtung bewegt haben. Schließlich gemäß Figur 10D detektiert Bereich 1041a noch Bereich 1041b Licht, ohne daß Bereich 1041a in der Zwischenzeit Licht detektierte. Das Strahlende hat sich somit eindeutig von der Abtastvorrichtung 1041 in die bisher bestimmte Richtung hinweg bewegt.

Eine derartige Abtastvorrichtung 1041 kann vorteilhafterweise dazu angewandt werden, festzustellen, in welche Richtung ein darauffallender Lichtstrahlende sich bewegt und/oder wann das Lichtstrahlende einen bestimmten Bereich verläßt.

Während die vorstehende Beschreibung sich titelgemäß auf Ausführungsbeispiele beschränkt, die unter die eingangs genannten Oberbegriffe "abtastendes Informationssystem" und "projizierendes Informationssystem" fallen, kann jedes besprochene Einzelmerkmal ihrer Offenbarung ebenfalls in einer Ausführung der eingangs durch vollinhaltliche Bezugnahme identifizerten Systemen, Vorrichtungen und Verfahren Anwendung finden. Die in der vorliegenden Anmeldung erwähnten Anmeldungen des gleichen Anmelders und/oder gleicher Erfinder sind als zusammengehöriges Erfindungskomplex anzusehen.

## Patentansprüche

1. Informationssystem (100) zum Zurverfügungstellen von Informationen mit
einem holographischen Element (820); und
einer optischen Abtastvorrichtung (841) zur Erfassung von auf ein Auge (810) fallendem Licht über das holographische Element, wobei
das holographische Element eine oder mehrere optische Markierungen (822) umfaßt, deren Lichtreflektionseigenschaften das Informationssystem mittels einer Photodetektorvorrichtung dazu heranziehen kann, einen Projektionswinkel einer optischen Projektionsvorrichtung (842) und/oder einer Lichtleitvorrichtung (844, 845) zu eichen.

2. Informationssystem gemäß Anspruch 1, wobei die optische Abtastvorrichtung In einem festen, vorbestimmten Winkelverhältnis zum holographischen Element steht.

3. Informationssystem gemäß Anspruch 1 oder 2, wobei das optischen Abtastvorrichtung Licht erfaßt, das von dem holographischen Element vor seinem Auftreffen auf das Auge gebeugt wird und nicht Ins Auge gelangt.

4. Informationssystem gemäß einem der vorhergehenden Ansprüche, wobei das optischen Abtastvorrichtung Licht erfaßt, das erst von dem Auge zurückreflektiert und dann vom holographischen Element gebeugt worden ist.

5. Informationssystem gemäß einem der vorhergehenden Ansprüche, wobei das holographische Element aus dem Gesichtsfeld des Auges stammendes Licht (132) lediglich bei einigen diskreten Wellenlängen im sichtbaren Bereich vor seinem Auftreffen auf das Auge zur Erfassung durch die optische Abtastvorrichtung beugt und von dem Auge zurückreflektiertes Licht (131) lediglich bei einer diskreten Wellenlänge im Infrarotbereich zur Erfassung durch die optische Abtastvorrichtung beugt.

6. Informationssystem gemäß einem der vorhergehenden Ansprüche, wobei das holographische Element aus dem Gesichtsfeld des Auges stammendes Licht (132) bei weniger als 20, weniger als 10 oder weniger als 5 diskreten Wellenlängen im sichtbaren Bereich entweder vor seinem Auftreffen auf das Auge oder nach seiner Rückstreuung aufgrund des Auges zur Erfassung durch die optische Abtastvorrichtung beugt.

7. Informationssystem gemäß einem der vorhergehenden Ansprüche, wobei das holographische Element aus dem Gesichtsfeld des Auges stammendes Licht (132) bei einer diskreten Wellenlänge im Infrarotbereich entweder vor seinem Auftreffen auf das Auge oder nach seiner Rückstreuung aufgrund des Auges zur Erfassung durch die optische Abtastvorrichtung beugt.

8. Informationssystem gemäß einem der vorhergehenden Ansprüche, wobei das holographische Element von dem Auge zurückreflektiertes Licht (131) lediglich bei einer diskreten Wellenlänge im Infrarotbereich zur Erfassung durch die optische Abtastvorrichtung beugt.

9. Informationssystem gemäß einem der vorhergehenden Ansprüche, wobei das holographische Element Licht einer oder einigen diskreten Wellenlängen beugt, bei der die optische Abtastvorrichtung eine starke Empfindlichkeit aufweist.

10. Informationssystem gemäß einem der vorhergehenden Ansprüche, wobei das holographische Element Licht bei einigen diskreten Wellenlängen derart beugt, daß das gebeugte Licht auf einen gemeinsamen Punkt (722) gelenkt wird, und der Einfallswinkel des Lichtes auf diesen Punkt einen eindeutigen, wahlweise auch wellenlängenunabhängigen Rückschluß auf den Einfallswinkel des Lichtes auf das holographische Element erlaubt.

11. Informationssystem gemäß einem der vorhergehenden Ansprüche, wobei das Informationssystem die Lichtreflektionseigenschaften der optischen Markierungen dazu heranzieht, einen Abtastwinkel der optischen Abtastvorrichtung und/oder einer Lichtleitvorrichtung zu eichen.

12. Informationssystem gemäß einem der vorhergehenden Ansprüche, mit einer optischen Projektionsvorrichtung (842), die Licht über das holographische Element in das Auge projiziert.

13. Informationssystem gemäß Anspruch 12, wobei das von der optischen Abtastvorrichtung erfaßte (631a) und das vor der optischen Projektionsvorrichtung projizierte (633) Licht in entgegengesetzter Richtung durch eine gemeinsame Lichtleitoptik (644, 645) läuft und derart durch die optische Abtastvorrichtung bzw. Projektionsvorrichtung fokusslerbar ist, daß ihre jeweiligen Strahlen den gleichen Pfad vom bzw. ins Auge beschreiben.

14. Informationssystem (100) zum Zurverfügungstellen von Informationen, mit
einem holographischen Element (820); und
einer optischen Projektionsvorrichtung (842) zur Projektion von Licht über das holographische Element in ein Auge (810), wobei
das holographische Element eine oder mehrere optische Markierungen (822) umfaßt, deren Lichtreflektionseigenschaften das Informationssystem mittels einer Photodetektorvorrichtung dazu heranziehen kann, einen Projektionswinkel der optischen Projektionsvorrichtung und/oder einer Lichtleitvorrichtung (844, 845) zu eichen.

15. Informationssystem gemäß einem der Ansprüche 12 bis 14, wobei die optische Projektionsvorrichtung Licht lediglich bei einer oder einigen diskreten Wellenlängen im sichtbaren Bereich und/oder bei einer Wellenlänge im Infrarotbereich projiziert.

16. Informationssystem gemäß einem der Ansprüche 12 bis 15, wobei das holographische Element die Wellenlängen des projizierten Lichtes beugt.

17. Informationssystem gemäß einem der Ansprüche 12 bis 16, wobei die optische Projektionsvorrichtung in einem festen, vorbestimmten Winkelverhältnis zum holographischen Element steht.

18. Informationssystem gemäß einem der Ansprüche 12 bis 17, wobei die optischen Markierungen **dadurch** erzeugt werden, daß spiegelnde Elemente bei der Erstellung des holographischen Elements derart im holographischen Element abgebildet werden, daß sie Licht einer oder mehreren Wellenlängen, das entsprechend dem vorbestimmten Winkelverhältnis zur optischen Projektionsvorrichtung auf das holographische Element fällt, entlang dem Einfallspfad zurückstrahlen.

19. Informationssystem gemäß Anspruch 18, wobei die Photodetektorvorrichtung einen Teilerspiegel (844) aufweist, der derart im Lichtstrahl der optischen Projektionsvorrichtung angeordnet ist, daß er einen Teil des Lichtes, das entgegen der Projektionsrichtung auf den Tellerspiegel trifft, in Richtung eines Photodetektors (941) lenkt, der in mindestens zwei konzentrisch umeinander liegenden Bereiche (941a, 941b) detektiert.

20. Informationssystem gemäß einem der vorhergehenden Ansprüche, wobei das holographische Element lichtbeugende Eigenschaften bei einer oder einigen diskreten Wellenlängen aufweist, die einer Spiegelung an der Konkavseite einer gemäß der Krümmung eines rotationssymmetrischen Ellipsoids ausgebildeten Fläche entsprechen.

21. Informationssystem gemäß einem der vorhergehenden Ansprüche, wobei das holographische Element lichtbeugende Eigenschaften bei einer oder einigen diskreten Wellenlängen aufweist, die einer Brechung an der Konkavseite einer gemäß der Krümmung eines rotationssymmetrischen Ellipsoids ausgebildeten Fläche entsprechen, welche Brechung einer Spiegelung an einer jeweiligen, um die Rotationsachse des Ellipsoids rotationssymmetrischen Kegelfläche entspricht, die am Ort der Brechung senkrecht zum Ellipsoid steht.

22. Verfahren zum Zurverfügungstellen von Informationen, bei dem
- ein holographisches Element (820) vor einem Auge (810) gelagert wird; und
- eine optische Abtastvorrichtung (841) das auf das Auge fallende Licht über das holographische Element erfaßt, wobei
das holographische Element mit einer oder mehreren optischen Markierungen (822) versehen wird, deren Lichtreflektionseigenschaften mittels einer Photodetektorvorrichtung dazu herangezogen werden können, einen Projektionswinkel einer optischen Projektionsvorrichtung (842) und/oder einer Lichtleitvorrichtung (844, 845) zu eichen.

23. Verfahren gemäß Anspruch 22, wobei die optische Abtastvorrichtung in einem festen, vorbestimmten Winkeiverhältnis zum holographischen Element steht.

24. Verfahren gemäß Anspruch 22 oder 23, wobei die optische Abtastvorrichtung Licht erfaßt, das von dem holographischen Element vor seinem Auftreffen auf das Auge gebeugt wird und nicht ins Auge gelangt.

25. Verfahren gemäß einem der Ansprüche 22 bis 24, wobei das optischen Abtastvorrichtung Licht erfaßt, das erst von dem Auge zurückreflektiert und dann vom holographischen Element gebeugt worden ist.

26. Verfahren gemäß einem der Ansprüche 22 bis 25, wobei das holographische Element aus dem Gesichtsfeld des Auges stammendes Licht (132) lediglich bei einigen diskreten Wellenlängen im sichtbaren Bereich vor seinem Auftreffen auf das Auge zur Erfassung durch die optische Abtastvorrichtung beugt und von dem Auge zurückreflektiertes Licht (131) lediglich bei einer diskreten Wellenlänge im Infrarotbereich zur Erfassung durch die optische Abtastvorrichtung beugt.

27. Verfahren gemäß einem der Ansprüche 22 bis 26, wobei das holographische Element aus dem Gesichtsfeld des Auges stammendes Licht (132) bei weniger als 20, weniger als 10 oder weniger als 5 diskreten Wellenlängen im sichtbaren Bereich entweder vor seinem Auftreffen auf das Auge oder nach seiner Rückstreuung aufgrund des Auges zur Erfassung durch die optische Abtastvorrichtung beugt.

28. Verfahren gemäß einem der Ansprüche 22 bis 27, wobei das holographische Element aus dem Gesichtsfeld des Auges stammendes Licht (132) bei einer diskreten Wellenlänge im Infrarotbereich entweder vor seinem Auftreffen auf das Auge oder nach seiner Rückstreuung aufgrund des Auges zur Erfassung durch die optische Abtastvorrichtung beugt.

29. Verfahren gemäß einem der Ansprüche 22 bis 28, wobei das holographische Element von dem Auge zurückreflektiertes Licht (131) lediglich bei einer diskreten Wellenlänge im Infrarotbereich zur Erfassung durch die optische Abtastvorrichtung beugt.

30. Verfahren gemäß einem der Ansprüche 22 bis 29, wobei das holographische Element Licht einer oder einigen diskreten Wellenlängen beugt, bei der die optische Abtastvorrichtung eine starke Empfindlichkeit aufweist.

31. Verfahren gemäß einem der Ansprüche 22 bis 30, wobei das holographische Element Licht bei einigen diskreten Wellenlängen derart beugt, daß das gebeugte Licht auf einen gemeinsamen Punkt (722) gelenkt wird, und der Einfallswinkel des Lichtes auf diesen Punkt einen eindeutigen, wahlweise auch wellenlängenunabhängigen Rückschluß auf den Einfallswinkel des Lichtes auf das holographische Element erlaubt.

32. Verfahren gemäß einem der Ansprüche 22 bis 31, wobei die Lichtreflektionselgenschaften der optischen Markierungen dazu herangezogen werden, einen Abtastwinkel der optischen Abtastvorrichtung und/oder einer Lichtleitvorrichtung zu eichen.

33. Verfahren gemäß einem der Ansprüche 22 bis 32, bei dem eine optische Projektionsvorrichtung (842) Licht über das holographische Element In das Auge projiziert.

34. Verfahren gemäß Anspruch 33, wobei das von der optischen Abtastvorrichtung erfaßte (631a) und das vor der optischen Projektionsvorrichtung projizierte (633) Licht In entgegengesetzter Richtung durch eine gemeinsame Lichtleitoptik (644, 645) läuft und derart durch die optische Abtastvorrichtung bzw. Projektionsvorrichtung fokusslerbar ist, daß ihre jeweiligen Strahlen den gleichen Pfad vom bzw. Ins Auge beschreiben.

35. Verfahren zum Zurverfügungstellen von Informationen, bei dem
- ein holographisches Element (820) vor einem Auge (810) gelagert wird; und
- eine optische Projektionsvorrichtung (842) Licht über das holographischen Element in das Auge projiziert, wobei
das holographische Element mit einer oder mehreren optischen Markierungen (822) versehen wird, deren Lichtreflektionseigenschaften mittels einer Photodetektorvorrichtung dazu herangezogen werden können, einen Projektionswinkel der optischen Projektionsvorrichtung und/oder einer Lichtleitvorrichtung (844, 845) zu eichen.

36. Verfahren gemäß einem der Ansprüche 33 bis 35, wobei die optische Projektionsvorrichtung Licht lediglich bei einer oder einigen diskreten Wellenlängen im sichtbaren Bereich und/oder bei einer Wellenlänge Im Infrarotbereich projiziert.

37. Verfahren gemäß einem der Ansprüche 33 bis 36, wobei das holographische Element die Wellenlängen des projizierten Lichtes beugt.

38. Verfahren gemäß einem der Ansprüche 33 bis 37, wobei die optische Projektionsvorrichtung In einem festen, vorbestimmten Winkelverhältnis zum holographischen Element steht.

39. Verfahren gemäß einem der Ansprüche 33 bis 38, wobei die optischen Markierungen **dadurch** erzeugt werden, daß spiegelnde Elemente bei der Erstellung des holographischen Elements derart im holographischen Element abgebildet werden, daß sie Licht einer oder mehreren Wellenlängen, das entsprechend dem vorbestimmten Winkelverhältnis zur optischen Projektionsvorrichtung auf das holographische Element fällt, entlang dem Einfallspfad zurückstrahlt werden.

40. Verfahren gemäß Anspruch 39, wobei die Photodetektorvorrichtung mit einem in mindestens zwei konzentrisch umeinander liegenden Bereiche (941a, 941b) detektierenden Photodetektor (941) und einem Teilerspiegel (844) versehen Ist, der derart im Lichtstrahl der optischen Projektionsvorrichtung angeordnet ist, daß er einen Teil des Lichtes, das entgegen der Projektionsrichtung auf den Teilerspiegel trifft, in Richtung des Photodetektors lenkt.

41. Verfahren gemäß einem der Ansprüche 22 bis 40, wobei das holographische Element lichtbeugende Eigenschaften bei einer oder einigen diskreten Wellenlängen aufweist, die einer Spiegelung an der Konkavseite einer gemäß der Krümmung eines rotationssymmetrischen Ellipsoids ausgebildeten Fläche entsprechen.

42. Verfahren gemäß einem der Ansprüche 22 bis 41, wobei das holographische Element lichtbeugende Eigenschaften bei einer oder einigen diskreten Wellenlängen aufweist, die einer Brechung an der Konkavseite einer gemäß der Krümmung eines rotationssymmetrischen Ellipsoids ausgebildeten Fläche entsprechen, welche Brechung einer Spiegelung an einer jeweiligen, um die Rotationsachse des Ellipsoids rotationssymmetrischen Kegelfläche entspricht, die am Ort der Brechung senkrecht zum Ellipsoid steht.

## Claims

1. An information system (100) for providing information, comprising:
a holographic element (820); and
an optical detection apparatus (841) for capturing, via said holographic element, light falling onto an eye (810), wherein
the holographic element encompasses one or more optical markings (822) whose light reflective properties can be exploited by the information system by means of a photodetection apparatus in order to calibrate a projection angle of an optical projection apparatus (842) and/or of a light guiding apparatus (844, 845).

2. The information system of claim 1, wherein the optical detection apparatus is situated in a predetermined, fixed angular relationship to the holographic element.

3. The information system of claim 1 or 2, wherein the optical detection apparatus captures light that is diffracted by the holographic element prior to its incidence onto the eye and does not enter the eye.

4. The information system in accordance with one of the preceding claims, wherein the optical detection apparatus captures light that is first reflected back from the eye and then diffracted by the holographic element.

5. The information system in accordance with one of the preceding claims, wherein the holographic element diffracts solely several discrete wavelengths of light (132) in the visible range originating from the field of view of the eye prior to its incidence onto the eye for capture via the optical detection apparatus and diffracts solely one discrete wavelength of light (131) in the infrared range reflected back from the eye for capture via the optical detection apparatus.

6. The information system in accordance with one of the preceding claims, wherein the holographic element diffracts less than 20, less than 10 or less than 5 discrete wavelengths of light (132) in the visible range originating from the field of view of the eye for capture via the optical detection apparatus either prior to its incidence onto the eye or after its backscattering due to the eye.

7. The information system in accordance with one of the preceding claims, wherein the holographic element diffracts one discrete wavelength of light (131) in the infrared range originating from the field of view of the eye for capture via the optical detection apparatus either prior to its incidence onto the eye or after its backscattering due to the eye.

8. The information system in accordance with one of the preceding claims, wherein the holographic element diffracts one discrete wavelength of light (131) in the infrared range reflected back from the eye for capture via the optical detection apparatus.

9. The information system in accordance with one of the preceding claims, wherein the holographic element diffracts light at one or several discrete wavelengths at which the optical detection apparatus exhibits high sensitivity.

10. The information system in accordance with one of the preceding claims, wherein the holographic element diffracts light at several discrete wavelengths such that the diffracted light is directed to a common point (722) and the angle of incidence of the light onto this point allows unambiguous conclusions to be drawn as regards the incident angle of the light onto the holographic element, optionally independent of the wavelength.

11. The information system in accordance with one of the preceding claims, wherein the information system exploits the light reflective properties of the optical markings in order to calibrate a detection angle of the optical detection apparatus and/or of a light guiding apparatus.

12. The information system in accordance with one of the preceding claims, comprising an optical projection apparatus (842) that projects light into the eye via the holographic element.

13. The information system in accordance with claim 12, wherein the light (631a) captured by the optical detection apparatus and the light (633) projected by the optical projection apparatus travels through common light guiding optics (644, 645) in opposite directions and is focusable via the optical detection apparatus / the projection apparatus such that their respective beams describe the same path from / onto the eye.

14. An information system (100) for providing information, comprising:
a holographic element (820); and
an optical projection apparatus (842) for projecting light onto an eye (810) via said holographic element, wherein
the holographic element encompasses one or more optical markings (822) whose light reflective properties can be exploited by the information system by means of a photodetection apparatus in order to calibrate a projection angle of the optical projection apparatus (842) and/or of a light guiding apparatus (844, 845).

15. The information system in accordance with one of claims 12 to 14, wherein the optical projection apparatus projects light solely at one or several discrete wavelengths in the visible range and/or at one wavelength in the infrared range.

16. The information system in accordance with one of claims 12 to 15, wherein the holographic element diffracts the wavelengths of the projected light.

17. The information system in accordance with one of claims 12 to 16, wherein the optical projection apparatus is situated in a predetermined, fixed angular relationship to the holographic element.

18. The information system in accordance with one of claims 12 to 17, wherein the optical markings are created by reproducing mirroring elements in the holographic element during creation of the holographic element such that they reflect light at one or more wavelengths that has fallen onto the holographic element in accordance with the predetermined angular relationship to the optical projection apparatus back along the path of incidence.

19. The information system in accordance with claim 18, wherein the photodetection apparatus comprises a beam splitter (844) that is situated in the light beam of the optical projection apparatus such that it directs a portion of the light that impinges on the beam splitter opposite to the projection direction in the direction of a photodetector (941) that detects in at least two regions (941a, 941b) situated concentrically around one another.

20. The information system in accordance with one of the preceding claims, wherein the holographic element exhibits, at one or several discrete wavelengths, light diffracting properties that correspond to a reflection on the concave side of a surface formed in accordance with the curvature of a rotationally symmetric ellipsoid.

21. The information system in accordance with one of the preceding claims, wherein the holographic element exhibits, at one or several discrete wavelengths, light diffracting properties that correspond to a diffraction on the concave side of a surface formed in accordance with the curvature of a rotationally symmetric ellipsoid, which diffraction corresponds to a reflection on a respective, cone surface that is rotationally symmetric to the rotational axis of the ellipsoid and that is perpendicular to the ellipsoid at the location of diffraction.

22. A method for providing information, in which
- a holographic element (820) is placed in front of an eye (810); and
- an optical detection apparatus (841) captures, via said holographic element, light falling onto the eye, wherein
said holographic element is provided with one or more optical markings (822) whose light reflective properties can be exploited by means of a photodetection apparatus in order to calibrate a projection angle of an optical projection apparatus (842) and/or of a light guiding apparatus (844, 845).

23. The information system of claim 22, wherein the optical detection apparatus is situated in a predetermined, fixed angular relationship to the holographic element.

24. The information system of claim 22 or 23, wherein the optical detection apparatus captures light that is diffracted by the holographic element prior to its incidence onto the eye and does not enter the eye.

25. The information system in accordance with one of claims 22 to 24, wherein the optical detection apparatus captures light that is first reflected back from the eye and then diffracted by the holographic element.

26. The information system in accordance with one of claims 22 to 25, wherein the holographic element diffracts solely several discrete wavelengths of light (132) in the visible range originating from the field of view of the eye prior to its incidence onto the eye for capture via the optical detection apparatus and diffracts solely one discrete wavelength of light (131) in the infrared range reflected back from the eye for capture via the optical detection apparatus.

27. The information system in accordance with one of claims 22 to 26, wherein the holographic element diffracts less than 20, less than 10 or less than 5 discrete wavelengths of light (132) in the visible range originating from the field of view of the eye for capture via the optical detection apparatus either prior to its incidence onto the eye or after its backscattering due to the eye.

28. The information system in accordance with one of claims 22 to 27, wherein the holographic element diffracts one discrete wavelength of light (131) in the infrared range originating from the field of view of the eye for capture via the optical detection apparatus either prior to its incidence onto the eye or after its backscattering due to the eye.

29. The information system in accordance with one of claims 22 to 28, wherein the holographic element diffracts one discrete wavelength of light (131) in the infrared range reflected back from the eye for capture via the optical detection apparatus.

30. The information system in accordance with one of claims 22 to 29, wherein the holographic element diffracts light at one or several discrete wavelengths at which the optical detection apparatus exhibits high sensitivity.

31. The information system in accordance with one of claims 22 to 30, wherein the holographic element diffracts light at several discrete wavelengths such that the diffracted light is directed to a common point (722) and the angle of incidence of the light onto this point allows unambiguous conclusions to be drawn as regards the incident angle of the light onto the holographic element, optionally independent of the wavelength.

32. The information system in accordance with one of claims 22 to 31, wherein the light reflective properties of the optical markings are exploited in order to calibrate a detection angle of the optical detection apparatus and/or of a light guiding apparatus.

33. The information system in accordance with one of claims 22 to 32, wherein an optical projection apparatus (842) projects light into the eye via the holographic element.

34. The information system in accordance with claim 33, wherein the light (631a) captured by the optical detection apparatus and the light (633) projected by the optical projection apparatus travels through common light guiding optics (644, 645) in opposite directions and is focusable via the optical detection apparatus / the projection apparatus such that their respective beams describe the same path from / onto the eye.

35. A method for providing information, in which
- a holographic element (820) is placed in front of an eye (810); and
- an optical projection apparatus (842) captures, via said holographic element, light falling onto the eye, wherein
said holographic element is provided with one or more optical markings (822) whose light reflective properties can be exploited by means of a photodetection apparatus in order to calibrate a projection angle of the optical projection apparatus and/or of a light guiding apparatus (844, 845).

36. The information system in accordance with one of claims 33 to 35, wherein the optical projection apparatus projects light solely at one or several discrete wavelengths in the visible range and/or at one wavelength in the infrared range.

37. The information system in accordance with one of claims 33 to 36, wherein the holographic element diffracts the wavelengths of the projected light.

38. The information system in accordance with one of claims 33 to 37, wherein the optical projection apparatus is situated in a predetermined, fixed angular relationship to the holographic element.

39. The information system in accordance with one of claims 33 to 38, wherein the optical markings are created by reproducing mirroring elements in the holographic element during creation of the holographic element such that they reflect light at one or more wavelengths that has fallen onto the holographic element in accordance with the predetermined angular relationship to the optical projection apparatus back along the path of incidence.

40. The information system in accordance with claim 39, wherein the photodetection apparatus is provided with a photodetector (941) that detects in at least two regions (941a, 941b) situated concentrically around one another and a beam splitter (844) that is situated in the light beam of the optical projection apparatus such that it directs a portion of the light that impinges on the beam splitter opposite to the projection direction in the direction of the photodetector.

41. The information system in accordance with one of claims 22 to 40, wherein the holographic element exhibits, at one or several discrete wavelengths, light diffracting properties that correspond to a reflection on the concave side of a surface formed in accordance with the curvature of a rotationally symmetric ellipsoid.

42. The information system in accordance with one of claims 22 to 41, wherein the holographic element exhibits, at one or several discrete wavelengths, light diffracting properties that correspond to a diffraction on the concave side of a surface formed in accordance with the curvature of a rotationally symmetric ellipsoid, which diffraction corresponds to a reflection on a respective, cone surface that is rotationally symmetric to the rotational axis of the ellipsoid and that is perpendicular to the ellipsoid at the location of diffraction.

## Revendications

1. Système d'information (100) destiné à fournir des informations à l'aide d'un élément holographique (820); et
d'un dispositif de balayage optique (841) prévu pour capter la lumière frappant un oeil (810) au moyen de l'élément holographique, dans lequel
ledit élément holographique comprend un ou plusieurs repères optiques (822) dont les propriétés de réflexion lumineuse peuvent servir par le biais d'un dispositif de photodétection à amener le système d'information à étalonner l'angle de projection d'un dispositif de projection optique (842) et / ou d'un dispositif de conduction de lumière (844, 845).

2. Système d'information selon la revendication 1, dans lequel le dispositif de balayage optique est disposé à un angle fixe, prédéfini par rapport à l'élément holographique.

3. Système d'information conformément aux revendications 1 ou 2, dans lequel le dispositif de balayage optique capte la lumière qui est diffractée par l'élément holographique avant de toucher l'oeil et ne parvient donc pas dans l'oeil.

4. Système d'information selon l'une des revendications précédentes, dans lequel le dispositif de balayage optique capte la lumière seulement après qu'elle a été réfléchie par l'oeil et diffractée ensuite par l'élément holographique.

5. Système d'information selon l'une des revendications précédentes, dans lequel l'élément holographique diffracte la lumière émanant du champ visuel de l'oeil (132) uniquement sur certaines longueurs d'onde discrètes dans la plage visible du spectre avant son incidence sur l'oeil pour être captée par le dispositif de balayage optique et qui diffracte la lumière réfléchie par l'oeil (131) seulement sur une longueur d'onde discrète dans la plage infrarouge du spectre pour être captée par le dispositif de balayage optique.

6. Système d'information selon l'une des revendications précédentes, dans lequel l'élément holographique diffracte la lumière émanant du champ visuel de l'oeil (132) sur moins de 20, de 10 ou de 5 longueurs d'onde discrètes dans la plage visible du spectre, soit avant son incidence sur l'oeil, soit après sa rétrodiffusion imputable à l'oeil pour être captée par le dispositif de balayage optique.

7. Système d'information selon l'une des revendications précédentes, dans lequel l'élément holographique diffracte la lumière émanant du champ visuel de l'oeil (132) seulement sur une longueur d'onde discrète dans la plage infrarouge du spectre, soit avant son incidence sur l'oeil, soit après sa rétrodiffusion imputable à l'oeil pour être captée par le dispositif de balayage optique.

8. Système d'information selon l'une des revendications précédentes, dans lequel l'élément holographique diffracte la lumière réfléchie par l'oeil (131) seulement sur une longueur d'onde discrète dans la plage infrarouge du spectre pour être captée par le dispositif de balayage optique.

9. Système d'information selon l'une des revendications précédentes, dans lequel l'élément holographique diffracte la lumière émise sur une ou quelques longueurs d'onde discrètes à laquelle ou auxquelles le dispositif de balayage optique présente une forte sensibilité.

10. Système d'information selon l'une des revendications précédentes, dans lequel l'élément holographique diffracte la lumière émise sur certaines longueurs d'onde discrètes de sorte que la lumière ainsi diffractée est guidée vers un point commun (722) et que l'angle d'incidence de la lumière sur ce point permet d'en déduire clairement, au choix aussi indépendamment de la longueur d'onde, l'angle d'incidence de la lumière sur l'élément holographique.

11. Système d'information selon l'une des revendications précédentes, dans lequel ledit système d'information exploite les propriétés de réflexion lumineuse des repères optiques pour étalonner l'angle de balayage du dispositif de balayage optique et / ou d'un dispositif de conduction de lumière.

12. Système d'information selon l'une des revendications précédentes, avec un dispositif de projection optique (842) qui diffuse la lumière dans l'oeil au moyen de l'élément holographique.

13. Système d'information selon la revendication 12, dans lequel la lumière captée par le dispositif de balayage optique (631a) et la lumière projetée en amont du dispositif de projection optique (633) traversent une optique conductrice de lumière commune (644, 645) en sens opposé et sont focalisables par le dispositif de balayage optique ou le dispositif de projection optique de manière à ce que leurs faisceaux respectifs décrivent le même trajet en provenance ou en direction de l'oeil.

14. Système d'information (100) destiné à fournir des informations à l'aide d'un élément holographique (820); et
d'un dispositif de projection optique (842) prévu pour diffuser la lumière dans un oeil (810) au moyen de l'élément holographique, dans lequel
ledit élément holographique comprend un ou plusieurs repères optiques (822) dont les propriétés de réflexion lumineuse peuvent servir par le biais d'un dispositif de photodétection à amener le système d'information à étalonner l'angle de projection d'un dispositif de projection optique (842) et / ou d'un dispositif de conduction de lumière (844, 845).

15. Système d'information selon l'une des revendications de 12 à 14, dans lequel le dispositif de projection optique diffuse la lumière émise uniquement sur une ou quelques longueurs d'onde discrètes dans la plage visible du spectre et / ou sur une longueur d'onde dans la plage infrarouge.

16. Système d'information selon l'une des revendications de 12 à 15, dans lequel l'élément holographique diffracte les longueurs d'onde de la lumière projetée.

17. Système d'information selon l'une des revendications de 12 à 16, dans lequel le dispositif de projection optique est aménagé à un angle fixe, prédéfini par rapport à l'élément holographique.

18. Système d'information selon l'une des revendications de 12 à 17, dans lequel les repères optiques sont générés du fait que des éléments réfléchissants reproduisent leur image dans l'élément holographique au moment de son élaboration de sorte qu'ils renvoient le long de son trajet d'incidence la lumière qui est émise sur une ou plusieurs longueurs d'onde et vient frapper l'élément holographique selon l'ange préétabli par rapport au dispositif de projection optique.

19. Système d'information selon la revendication 18, dans lequel le dispositif de photodétection présente un miroir séparateur (844) qui est disposé dans le faisceau lumineux du dispositif de projection optique de manière à ce qu'il dirige une partie de la lumière qui parvient sur ledit miroir séparateur dans le sens opposé à la projection vers un photodétecteur (941) dont la portée couvre au moins deux zones concentriques adjacentes (941a, 941b).

20. Système d'information selon l'une des revendications précédentes, dans lequel l'élément holographique présente des propriétés diffractives sur une ou quelques longueurs d'onde discrètes qui correspondent à la réflexion sur la face concave d'une surface configurée d'après la courbure d'un ellipsoïde à symétrie de révolution.

21. Système d'information selon l'une des revendications précédentes, dans lequel l'élément holographique présente des propriétés diffractives sur une ou quelques longueurs d'onde discrètes qui correspondent à la réfraction sur la face concave d'une surface configurée d'après la courbure d'un ellipsoïde à symétrie de révolution, ladite réfraction étant telle qu'elle correspond à la réflexion sur la surface conique respective à symétrie de révolution autour de l'axe de rotation de l'ellipsoïde et disposée à la perpendiculaire dudit ellipsoïde à l'endroit de la réfraction.

22. Procédé de mise à disposition d'informations dans lequel
- un élément holographique (820) est monté devant un oeil (810) ; et
- un dispositif de balayage optique (841) capte la lumière incidente à la surface de l'oeil au moyen de l'élément holographique, dans lequel
l'élément holographique est pourvu d'un ou plusieurs repères optiques (822) dont les propriétés de réflexion lumineuse peuvent être exploitées au moyen d'un dispositif de photodétection pour étalonner l'angle de projection d'un dispositif de projection optique (842) et / ou d'un dispositif de conduction de lumière (844, 845).

23. Procédé selon la revendication 22, dans lequel le dispositif de balayage optique est disposé à un angle fixe, prédéfini par rapport à l'élément holographique.

24. Procédé selon les revendications 22 ou 23, dans lequel le dispositif de balayage optique capte la lumière qui est diffractée par l'élément holographique avant de frapper l'oeil et ne parvient donc pas dans l'oeil.

25. Procédé selon l'une des revendications de 22 à 24, dans lequel le dispositif de balayage optique capte la lumière seulement après qu'elle a été réfléchie par l'oeil et diffractée ensuite par l'élément holographique.

26. Procédé selon l'une des revendications de 22 à 25, dans lequel l'élément holographique diffracte la lumière émanant du champ visuel de l'oeil (132) uniquement sur certaines longueurs d'onde discrètes dans la plage visible du spectre avant son incidence sur l'oeil pour être captée par le dispositif de balayage optique et qui diffracte la lumière réfléchie par l'oeil (131) seulement sur une longueur d'onde discrète dans la plage infrarouge du spectre pour être captée par le dispositif de balayage optique.

27. Procédé selon l'une des revendications de 22 à 26, dans lequel l'élément holographique diffracte la lumière émanant du champ visuel de l'oeil (132) sur moins de 20, de 10 ou de 5 longueurs d'onde discrètes dans la plage visible du spectre, soit avant son incidence sur l'oeil, soit après sa rétrodiffusion imputable à l'oeil pour être captée par le dispositif de balayage optique.

28. Procédé selon l'une des revendications de 22 à 27, dans lequel l'élément holographique diffracte la lumière émanant du champ visuel de l'oeil (132) sur une longueur d'onde discrète dans la plage infrarouge du spectre, soit avant son incidence sur l'oeil, soit après sa rétrodiffusion imputable à l'oeil pour être captée par le dispositif de balayage optique.

29. Procédé selon l'une des revendications de 22 à 28, dans lequel l'élément holographique diffracte la lumière réfléchie par l'oeil (131) seulement sur une longueur d'onde discrète dans la plage infrarouge du spectre pour être captée par le dispositif de balayage optique.

30. Procédé selon l'une des revendications de 22 à 29, dans lequel l'élément holographique diffracte la lumière émise sur une ou quelques longueurs d'onde discrètes à laquelle ou auxquelles le dispositif de balayage optique présente une forte sensibilité.

31. Procédé selon l'une des revendications de 22 à 30, dans lequel l'élément holographique diffracte la lumière émise sur certaines longueurs d'onde discrètes de sorte que la lumière ainsi diffractée est guidée vers un point commun (722) et que l'angle d'incidence de la lumière sur ce point permet d'en déduire clairement, au choix aussi indépendamment de la longueur d'onde, l'angle d'incidence de la lumière sur l'élément holographique.

32. Procédé selon l'une des revendications de 22 à 31, dans lequel les propriétés de réflexion lumineuse des repères optiques sont exploitées pour étalonner l'angle de balayage du dispositif de balayage optique et / ou d'un dispositif de conduction de lumière.

33. Procédé selon l'une des revendications de 22 à 32 dans lequel un dispositif de projection optique (842) diffuse la lumière dans l'oeil au moyen de l'élément holographique.

34. Procédé selon la revendication 33, dans lequel la lumière captée par le dispositif de balayage optique (631a) et la lumière projetée en amont du dispositif de projection optique (633) traversent une optique conductrice de lumière commune (644, 645) en sens opposé et sont focalisables par le dispositif de balayage optique ou le dispositif de projection optique de manière à ce que leurs faisceaux respectifs décrivent le même trajet en provenance ou en direction de l'oeil.

35. Procédé de mise à disposition d'informations dans lequel
- un élément holographique (820) est monté devant un oeil (810) ; et
- un dispositif de projection optique (842) diffuse de la lumière dans l'oeil (810) au moyen de l'élément holographique, dans lequel
l'élément holographique est pourvu d'un ou plusieurs repères optiques (822) dont les propriétés de réflexion lumineuse peuvent être exploitées au moyen d'un dispositif de photodétection pour étalonner l'angle de projection d'un dispositif de projection optique (842) et / ou d'un dispositif de conduction de lumière (844, 845).

36. Procédé selon l'une des revendications de 33 à 35, dans lequel le dispositif de projection optique diffuse la lumière émise uniquement sur une ou quelques longueurs d'onde discrètes dans la plage visible du spectre et / ou sur une longueur d'onde dans la plage infrarouge.

37. Procédé selon l'une des revendications de 33 à 36, dans lequel l'élément holographique diffracte les longueurs d'onde de la lumière projetée.

38. Procédé selon l'une des revendications de 33 à 37, dans lequel le dispositif de projection optique est aménagé à un angle fixe, prédéfini par rapport à l'élément holographique.

39. Procédé selon l'une des revendications de 33 à 38, dans lequel les repères optiques sont générés du fait que des éléments réfléchissants reproduisent leur image dans l'élément holographique au moment de son élaboration de sorte qu'ils renvoient le long de son trajet d'incidence la lumière qui est émise sur une ou plusieurs longueurs d'onde et vient frapper l'élément holographique selon l'ange préétabli par rapport au dispositif de projection optique.

40. Procédé selon la revendication 39, dans lequel le dispositif de photodétection est muni d'un photodétecteur (941) dont la portée couvre au moins deux zones concentriques adjacentes (941a, 941b) et d'un miroir séparateur (844) qui est disposé dans le faisceau lumineux du dispositif de projection optique de manière à ce qu'il dirige une partie de la lumière qui parvient sur ledit miroir séparateur dans le sens opposé à la projection.

41. Procédé selon l'une des revendications de 22 à 40, dans lequel l'élément holographique présente des propriétés diffractives sur une ou quelques longueurs d'onde discrètes qui correspondent à la réflexion sur la face concave d'une surface configurée d'après la courbure d'un ellipsoïde à symétrie de révolution.

42. Procédé selon l'une des revendications de 22 à 41, dans lequel l'élément holographique présente des propriétés diffractives sur une ou quelques longueurs d'onde discrètes qui correspondent à la réfraction sur la face concave d'une surface configurée d'après la courbure d'un ellipsoïde à symétrie de révolution, ladite réfraction étant telle qu'elle correspond à la réflexion sur la surface conique respective à symétrie de révolution autour de l'axe de rotation de l'ellipsoïde et disposée à la perpendiculaire dudit ellipsoïde à l'endroit de la réfraction.
